# EUROPEAN PATENT SPECIFICATION

(11) **EP 2 175 035 B1**
(45) Date of publication and mention of the grant of the patent: **16.04.2014**
(21) Application number: 08791203.6
(22) Date of filing: 16.07.2008
(51) Int. Cl.: C12Q 1/68, C12N 15/09

(54) **METHOD FOR OBTAINING INFORMATION ON BIOLOGICAL RHYTHM BY USING HAIR**
VERFAHREN ZUR GEWINNUNG VON BIORHYTHMUS-INFORMATIONEN ANHAND DER HAARE
PROCÉDÉ D'OBTENTION D'INFORMATIONS SUR LE RYTHME BIOLOGIQUE À L'AIDE DE POILS

(30) Priority: 25.07.2007 JP 2007193477
(43) Date of publication of application: 14.04.2010
(73) Proprietor: Sony Corporation, Tokyo 108-0075 (JP)
(72) Inventor: YAMAMOTO, Takuro, Tokyo 108-0075 (JP); AKASHI, Makoto, Saga-shi Saga 840-8502 (JP); YAMASHITA, Shiko, Tokyo 108-0075 (JP); ABE, Tomoteru, Tokyo 108-0075 (JP); NAKAGAWA, Kazuhiro, Tokyo 108-0075 (JP); SOMA, Haruhiko, Tokyo 108-0075 (JP); NODE, Koichi, Saga-shi Saga 840-8502 (JP); KISHII, Noriyuki, Tokyo 108-0075 (JP); HAYAKAWA, Tomohiro, Tokyo 108-0075 (JP)
(74) Representative: Sexton, Jane Helen
(86) International application number: PCT/JP2008/062808
(87) International publication number: WO 2009/014036

(56) References cited:
- JP-A- 2007 020 559
- BROWN S.A. ET AL.: "The Period Length of Fibroblast Circadian Gene Expression Varies Widely among Human Individuals" PLOS BIOLOGY, vol. 3, 2005, pages 1813-1818, XP002603037
- TAKIMOTO MIEKO ET AL: "Daily expression of clock genes in whole blood cells in healthy subjects and a patient with circadian rhythm sleep disorder." AMERICAN JOURNAL OF PHYSIOLOGY. REGULATORY, INTEGRATIVE AND COMPARATIVE PHYSIOLOGY NOV 2005 LNKD- PUBMED:15961535, vol. 289, no. 5, November 2005 (2005-11), pages R1273-R1279, XP002603039 ISSN: 0363-6119
- KIM SUNG JAE ET AL: "Gene expression in head hair follicles plucked from men and women." ANNALS OF CLINICAL AND LABORATORY SCIENCE SPRING 2006 LNKD- PUBMED:16682506, vol. 36, no. 2, April 2006 (2006-04), pages 115-126, XP002603038 ISSN: 0091-7370
- AKASHI MAKOTO ET AL: "Noninvasive method for assessing the human circadian clock using hair follicle cells." PROCEEDINGS OF THE NATIONAL ACADEMY OF SCIENCES OF THE UNITED STATES OF AMERICA 31 AUG 2010 LNKD- PUBMED:20798039, vol. 107, no. 35, 31 August 2010 (2010-08-31), pages 15643-15648, XP002603040 ISSN: 1091-6490
- LAMONT ET AL: "From circadian clock gene expression to pathologies" SLEEP MEDICINE, ELSEVIER, AMSTERDAM, NL LNKD- DOI:10.1016/J.SLEEP.2006.11.002, vol. 8, no. 6, 28 March 2007 (2007-03-28), pages 547-556, XP022186173 ISSN: 1389-9457
- BROWN S.A. ET AL.: 'The Period Length of Fibroblast Circadian Gene Expression Varies Widely among Human Individuals' PLOS BIOLOGY vol. 3, 2005, pages 1813 - 1818, XP008130573
- YAMAMOTO T. ET AL.: 'Transcriptional oscillation of canonical clock genes in mouse peripheral tissues' BMC MOLECULAR BIOLOGY vol. 5, no. 1, 2004, page 18, XP021002729
- TOMOKO U. ET AL.: 'Shi Kosa Jokaku kara Fukujin Hishitsu o Kaishita Zenshin eno Jikoku Signal Dentatsu' EXPERIMENTAL MEDICINE vol. 24, 2006, pages 472 - 478
- KIMIJIMA T. ET AL.: 'Mohatsu kara no DNA Kata Bunseki' REPORTS OF NATIONAL RESEARCH INSTITUTE OF POLICE SCIENCE, RESEARCH ON FORENSIC SCIENCE vol. 45, 1992, page 32, XP008118315
- TIE J. ET AL.: 'DOP-PCR-ho o Mochiita Hito Mohatsu to Tsume kara no DNA Takei no Kenshutsu' DNA POLYMORPHISM vol. 13, 2005, pages 197 - 200

## Description

### Technical Field

This invention relates to a method for obtaining information on a biorhythm. More specifically, the present invention is concerned with a method for obtaining information especially on a circadian rhythm based on variations over time in an expression level of a clock gene in a hair follicle cell as defined in the claims.

### Background Art

Various biological responses (which can also be called "physiological processes") in an individual organism are each known to show a rhythm that self-sustainably fluctuates (oscillates) in cycle. This rhythm is called "biorhythm." In particular, a "circadian rhythm" which has a periodicity of about a day is considered to widely govern biological responses (physiological processes) such as sleep-wake cycles and daily fluctuations in body temperature, blood pressure and hormonal secretions. A circadian rhythm has also been revealed to also take part in daily fluctuations in mental and physical activities, locomotor ability and drug sensitivity.

A regulatory center (which is also called "central clock") for a biorhythm resides in the suprachiasmatic nucleus of the hypothalamus. In each peripheral tissue, an independently-oscillating "peripheral clock" also resides. As several circadian rhythm cycles can also be detected in cultured cells, the function of a peripheral clock is considered to be found in each individual cell. It is considered that, while the peripheral clocks in each tissue or cells independently oscillate, these oscillations are synchronized and unified under the influence of the central clock, thereby expressing and regulating a biorhythm.

In the regulation of a biorhythm, a class of genes called "clock genes" take part. These clock genes function as "biological clocks" by causing their expression to fluctuate (oscillate) autonomically. The clock genes govern various biological responses (physiological processes), such as those described above, by regulating the expression of various genes in other classes.

A polymorphism or variation in clock genes has been revealed to become a cause of onset of a cancer, diabetes, vascular disease, neurodegenerative disease, or the like. In recent years, the involvement of a polymorphism or variation in clock genes has also been indicated as a cause of onset with respect to neurodegenerative diseases such as bipolar disorder and depression, and a treatment method has been used in an attempt to reset through exposure to light a biological clock modulated by a polymorphism or variation.

On the other hand, for example, sleep-wake cycles are subjected not only to autonomic regulations by biological clocks but also to restrictions through life in society. By a variation in daily bedtime or wake-up time, a rhythm shift (phase shift) may take place between "a sleep-wake cycle in real life" and "a sleep-wake cycle by biological clocks." Such a rhythm shift is considered to become a cause of a so-called "jet lag" or a sleep disorder, or of such a mental disease as described above.

Further, the use of a biorhythm has also begun in an attempt to maximize the effects of drug treatment. Due to a circadian rhythm of a molecule as a target of a drug (drug target molecule) or an enzyme (drug-metabolizing enzyme) that metabolizes, the treatment effect of the drug is also considered to undergo daily fluctuations. A concept of "chronotherapy" has, therefore, been proposed that attempts to maximize the effect of treatment by setting an optimal dosage time for each drug.

As more familiar subjects of research, the use of the daily rhythms of mental or physical activities or locomotor ability has begun to study the time of activity that draws out one's ability to the maximum in learning or training and the time of meal ingestion that readily (or hardly) causes a weight gain.

From the foregoing, the accurate ascertainment of a biorhythm governed by biological clocks is believed to be very beneficial for the prevention of various diseases, the improvement of poor physical conditions such as time lag, the realization of chronotherapy, the exhibition of one's own ability, dieting, and the like.

PCT Patent Publication No. WO2004/012128 discloses a method for estimating a biological clock based on assay data of gene expression product in a standard sample collected from an individual organism. According to this biological clock estimation method, a molecular clock table for the estimation of the biological clock is prepared based on expression levels of the gene expression product (specifically, mRNA). It is, however, to be noted that no specific collected tissue (or cells) or assay target gene is described in PCT Patent Publication No. WO2004/012128.

BMC Molecular Biology 5:18 (2004 Oct 9) describes that in an experiment using mice, circadian rhythms of clock genes were detected in peripheral tissues of the heart, lungs, liver, stomach, spleen, kidneys, testicles and the like. It is, however, to be noted that no discussion is made on hair follicle cells in BMC Molecular Biology 5:18 (2004 Oct 9).

American Journal of Pathology 158(5):1793-801 (2001 May) describes that circadian rhythms of clock genes were detected in the skin or tunica mucosa oris collected from human subjects.

PLoS Biology 3(10):e338 (2005 Oct, Epub 2005 Sep 27) describes the results of an expression analysis of clock genes in fibroblasts by culturing and proliferating fibroblasts collected by skin biopsy from human subjects and introducing a reporter gene fused with a clock gene promoter. Further, American Journal of Pathology 158(5):1793-801 (2001 May) describes that by a similar procedure, circadian rhythms of clock genes in keratinocytes were detected by culturing and proliferating keratinocytes adhering to a hair root portion of a hair plucked off from a human subject.

The biological clock estimation method disclosed in PCT Patent Publication No. WO2004/012128 is a method based on expression levels of mRNA in a standard sample collected from an individual organism. In PCT Patent Publication No. WO2004/012128, however, no specific collected tissue (or cells) or assay target gene is described. Because circadian rhythms of clock genes can also be detected in peripheral tissues of the heart, lungs, liver, stomach, spleen, kidneys, testicles and the like (see BMC Molecular Biology 5:18 (2004 Oct 9)), a method that checks expression levels of clock genes in their peripheral tissues may also be contemplated. However, the collection of these tissues is high in invasiveness to tested individuals so that such a method is not considered to be practical when its application to human subjects is taken into view.

Accordingly, the method that checks expression levels of clock genes in leukocytes collected by blood collection has been conventionally adopted. With this method, however, physical affliction is also entailed upon blood collection. Moreover, an analysis of gene expressions over time requires to collect blood at intervals of several hours and hence to force each human subject to stay in a medical institution. For an assayer, on the other hand, there are problems that time and labor are needed for the isolation of leukocytes from collected blood and the assayer is exposed to a risk of infection from blood. In addition, there is also a potential problem that in the course of the isolation work, the expression of genes in leukocytes may vary due to stimulations caused as a result of the work, thereby possibly making it difficult to perform an accurate assay of expression levels.

As a method that may replace the method making use of leukocytes, a method that uses more-readily collectable skin or tunica mucosa oris may be contemplated (see American Journal of Pathology 158(5):1793-801 (2001 May)). When skin is used, however, punching of cutaneous tissues under topical anesthesia is needed so that this method is not necessarily considered to be a low-invasive method.

On the other hand, the method that uses tunica mucosa oris can limit invasiveness to an extremely low level. Tunica mucosa oris has already been used also in genotyping that uses a genome extracted from tunica mucosa oris. When it is desired to extract mRNA for the purpose of an analysis of a gene expression, however, a problem arises in that mRNA is prone to degradation.

Upon extracting mRNA from a biological tissue, an operation is generally needed to prevent degradation of mRNA. When it is desired to extract mRNA from tunica mucosa oris, however, there is a problem that, even if the above-described operation is conducted, the degradation of mRNA tends to take place due to an RNA-degrading enzyme (RNase) abundantly existing in saliva and the assay results available in the quantification of a gene expression become very unstable.

Takimoto et al. (2005) Am J Physiol Regul Integr Comp Physiol. 289, R1273-R1279 discloses attempts to elucidate the relationship between the expression of clock genes in whole blood cells and the clinical features characteristic of circadian rhythm sleep disorder.

Kim et al. (2006) Annals of Clinical & Laboratory Science, 36, 115-125 discloses that RNA of sufficient quality and quantity to be used in microarray hybridizations can be obtained from as little a single plucked human hair follicle.

A primary object of the present invention is, therefore, to newly provide a simple and low-invasive method for obtaining information on a biorhythm of an individual organism.

### Disclosure of the Invention

To achieve the above-described object, the present invention provides a method for obtaining information on a biorhythm of an individual organism based on variations over time in an expression level of a clock gene wherein:
(a) the clock gene is the Per3 clock gene described in Sequence ID No: 1; and
(b) Per3 levels are measured directly in a hair follicle cell adhering to a hair root portion of a body hair plucked from the individual organism by extracting RNA from the hair follicle cell.

In this method, the information on a biorhythm may also be obtained from the expression level of at least one additional clock gene.

The at least one additional clock gene is selected from the following genes or homologs thereof (1) to (6):
(1) Per2 gene described in Sequence ID NO: 3 or a homolog thereof,
(2) Bmal1 gene described in Sequence ID NO: 5 or a homolog thereof,
(3) Npas2 gene described in Sequence ID NO: 7 or a homolog thereof,
(4) Nr1d1 gene described in Sequence ID NO: 9 or a homolog thereof,
(5) Nrld2 gene described in Sequence ID NO: 11 or a homolog thereof, and
(6) Dbp gene described in Sequence ID NO: 13 or a homolog thereof.

In this method, an expression level fluctuation curve indicating the variations over time in the expression level for the or each clock gene can be used as a molecular clock table for the estimation of the biorhythm.

A phase shift of the biorhythm of the individual organism can be detected by preparing this molecular clock table a plurality of times about the individual organism and collating the resulting molecular clock tables with each other. By collating an expression level of the or each clock gene at a predetermined time with the molecular clock table, a phase shift of the biorhythm of the individual organism can also be detected.

The term "hair follicle cell" as used herein widely encompasses therein a class of cells adhering to a hair root portion of a body hair drawn out when the body hair is plucked off. More specifically, cells forming an inner root sheath, outer root sheath and papilla are included. It is to be noted that no particular limitation is imposed on the region of a body hair to be plucked off for obtaining hair follicle cells and that, when the present invention is applied to a human subject, for example, a hair, beard, arm hair, leg hair or the like can be used.

Further, the term "individual organism," which is a target in the present invention for obtaining information on a biorhythm, widely includes mice, rats, monkeys and the like in addition to human subjects, and no particular limitation is imposed thereon insofar as the individual organism is an animal having body hair. When an animal other than human is chosen as a target organism, a target animal homolog of the human clock gene described in Sequence ID NO: 1, 3, 5, 7, 9, 11 or 13 is used.

By the present invention, a method for obtaining information on a biorhythm of an individual organism in a simple and low-invasive manner can be provided.

### Brief Description of the Drawings

[FIG. 1]

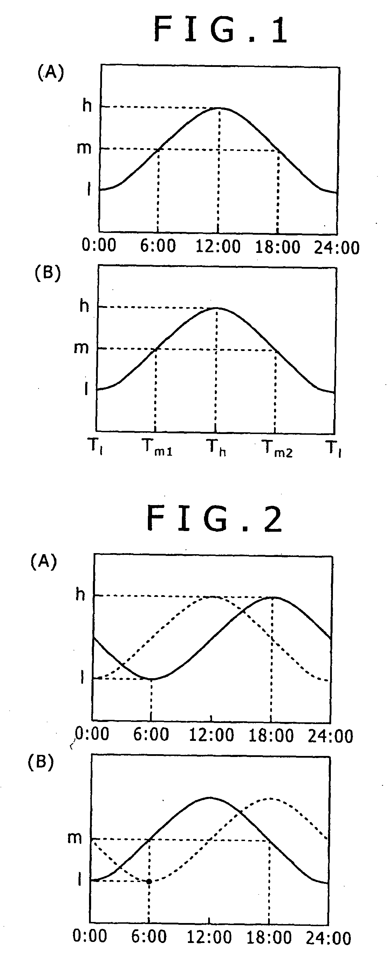

   FIG. 1 diagrammatically illustrates by way of example variations over time in the expression level of a clock gene.
[FIG. 2]
   FIG. 2 diagrammatically illustrates phase variations of the expression level fluctuation curves of the clock gene.
[FIG. 3]

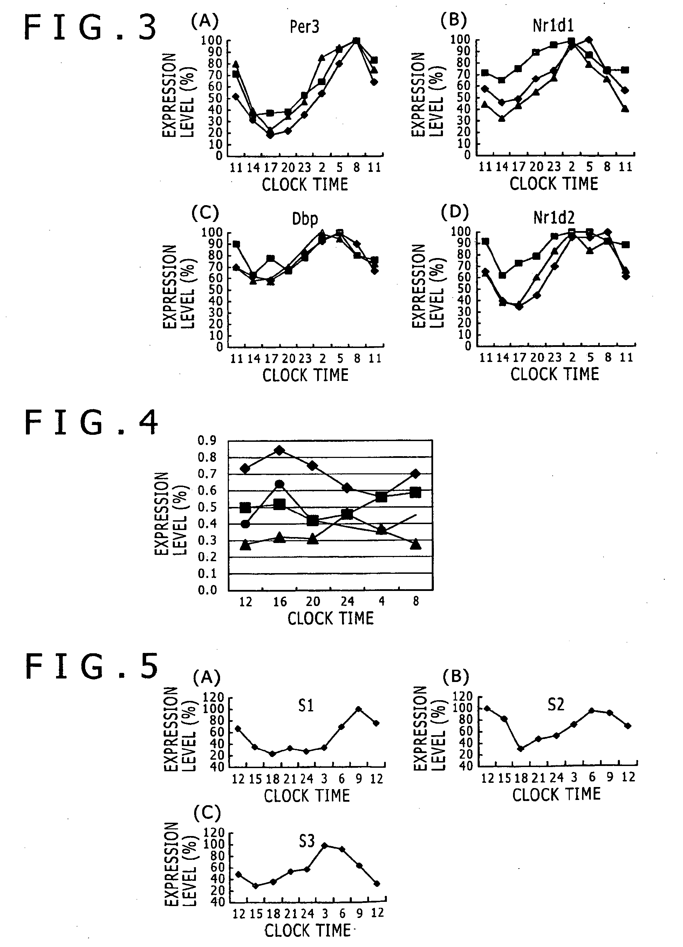

   FIG. 3 diagrammatically illustrates variations over time in the expression levels of clock genes in hair follicle cells (twenty hairs). (A) Per3 gene, (B) Nrld1 gene, (C) Dbp gene, and (D) Nrld2 gene.
[FIG. 4]
   FIG. 4 diagrammatically illustrates variations over time in the expression levels of clock genes in hair follicle cells (ten hairs).
[FIG. 5]
   FIG. 5 diagrammatically illustrates variations over time in the expression levels of Per3 gene in hair follicle cells (five hairs).
[FIG. 6]

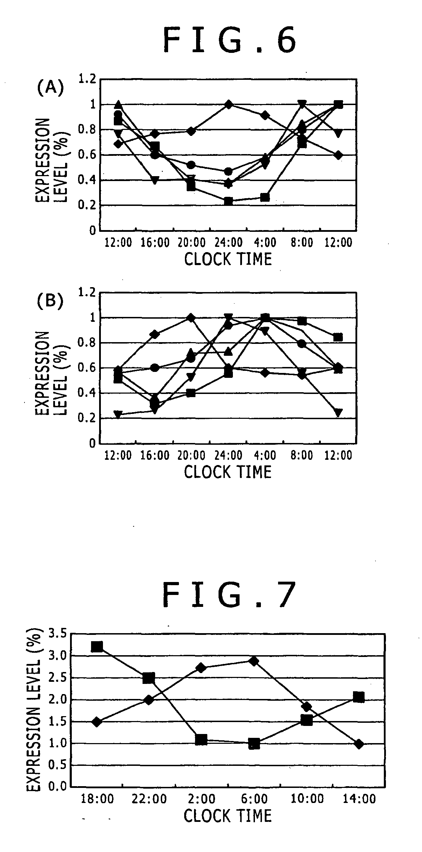

   FIG. 6 diagrammatically illustrates variations over time in the expression levels of clock genes in hair follicle cells (beards). (A) five beards, and (B) three beards.
[FIG. 7]
   FIG. 7 diagrammatically illustrates variations over time in the expression levels of clock genes in hair follicle cells (twenty arm hairs).

### Best Modes for Carrying Out the Invention

To establish a method for obtaining information on a biorhythm of an individual organism in a simple and low-invasive manner, the present inventors focused attention on hair follicle cells as a biological tissue collectable with extremely-low invasiveness.

Like tunica mucosa oris, hair follicle cells are employed in genotyping or the like that uses a genome. However, no attempt has heretofore been made to detect an expression of a clock gene directly from hair follicle cells adhering to a hair root portion of a plucked body hair. As its reason, it was once considered that hair follicle cells adhering to a hair root portion were too small in number to acquire RNA in any amount sufficient for the analysis of a gene expression. As another reason, it was considered difficult to extract RNA with quality sufficient for the analysis of an expression due to an RNA-degrading enzyme (RNase) abundantly existing in sweat.

For the resolution of such quantitative and qualitative problems of RNA, it may be contemplated to conduct an analysis of an expression of a clock gene in hair follicle cells after once culturing and proliferating hair follicle cells adhering to a hair root portion of a plucked hair as described in PLoS Biology 3(10):e338 (2005) Oct, Epub 2005 Sep 27).

As a peripheral clock is also found in each individual cell, several circadian rhythm cycles can still be detected even in such cultured hair follicle cells. However, the circadian rhythms so detected are rhythms produced by the hair follicle cells in the state that they had been cultured subsequent to their isolation from an organism and do not reflect a biorhythm synchronized and unified throughout the organism, because as already described, a biorhythm is expressed and regulated as a result of synchronization and unification of a central clock and peripheral clocks in an organism.

The present inventors, therefore, tried to detect a biorhythm by conducting an analysis of an expression of a clock gene directly with respect to a hair follicle cell adhering to a hair root portion of a plucked body hair. As a result, despite such a concern about the quantity and quality of RNA as described above, the present inventors succeeded in detecting an expression level of a clock gene in the hair follicle cell, and further, newly found that the expression level of the clock gene in the hair follicle cell shows a circadian rhythm, leading to the completion of the present invention.

Described specifically, the present invention relates to a method for obtaining information on a biorhythm of an individual organism based on variations over time in the expression level of a clock gene in a hair follicle cell of the individual organism.

In the present invention, no particular limitation is imposed on the collection region of hair follicle cells and, when the present invention is applied to human subjects, for example, hairs, beards, arm or leg hairs, or the like can be used. When the quantification of a gene expression is to be conducted over time, the collection of hair follicle cells is conducted a plurality of time at intervals of several hours. In doing so, it is desired to conduct the respective collections in regions as close as possible to each other to reduce variations in assay conditions.

The number of body hairs to be plucked off in each collection can be, in the case of human subjects, from 5 to 10 for hairs, from 3 to 5 for beards, and from 10 to 20 for arm hairs. The use of hairs, beards or arm hairs as many as the above-mentioned corresponding number or greater makes it possible to extract RNA in an amount sufficient for the quantification of an expression of a clock gene.

The extraction of RNA from hair follicle cells can be conducted by a method known to date, and for example, a commercially-available, RNA extraction kit for trace samples can be used. A reverse transcription reaction (cDNA synthesis) of the extracted RNA can also be conducted by a method known to date, and a commercially-available reverse transcriptase can be used. For the quantification of a gene expression, a real-time PCR assay which permits high-accuracy quantification is in common use at present. In the present invention, this real time PCR assay can also be suitably adopted, and further, various gene expression quantification methods can also be adopted.

Because hair follicle cells which can be collected in a simple and low-invasive manner are used in the method according to the present invention as described above, the physical load on each human subject can be extremely lightened. Different from the existing method that uses leukocytes, the method of the present invention does not require any cumbersome operation so that the results can be obtained in a short time. According to the method of the present invention, the expression level of a gene, therefore, does not vary in the course of a treatment operation of a sample.

As hair follicle cells not subjected to a culturing step are used in the method according to the present invention, the expression level of a clock gene in each hair follicle cell directly reflects the biorhythm of the tested individual at the time of plucking of the body hair. It is, therefore, possible to accurately estimate the biorhythm of the tested individual by conducting the collection of hair follicle cells over time and assaying variations in expression level.

In the present invention, the clock gene as a target of quantification of an expression is the Per3 gene (see SEQ ID NOS: 1 and 2). Other clock genes can be any one of a class of clock genes identified to date. Representative clock genes include Per2 gene (see SEQ ID NOS: 3 and 4), Bmal1 gene (see SEQ ID NOS: 5 and 6), Npas2 gene (see SEQ ID NOS: 7 and 8), Nrld1 gene (see SEQ ID NOS: 9 and 10), Nrld2 gene (see SEQ ID NOS: 11 and 12), Dbp gene (see SEQ ID NOS: 13 and 14), and the like.

It has become evident that among these, particularly Per3 gene undergoes largest daily fluctuations in expression level in a hair follicle cell and allows to assay variations over time in expression level more clearly compared with the other genes. The biorhythm of a tested individual can, therefore, be estimated more accurately provided that the method is conducted based on variations over time in the expression level of Per3 gene.

Plural ones of the above-described clock genes may be chosen as assay targets. A more accurate estimation is feasible by conducting the estimation of a biorhythm of a tested individual based on the expression levels of plural clock genes showing different daily fluctuation patterns.

It is to be noted that representative clock genes include Per1 gene in addition to the above-described genes. With respect to Per1 gene, however, it has been revealed, by an experiment using mice, that its expression level fluctuates by stress loads (see J. Biol Chem 280(51):42036-43 (2005 Dec 23, Epub 2005 Oct 24)). As an unexpected variation in expression level may hence occur under a stress to a tested individual at the time of plucking of a body hair, Per1 gene is considered to be unsuitable as an assay target gene in the present invention.

FIG. 1 diagrammatically illustrates variations over time in an expression level of a clock gene in a hair follicle cell. Each diagram shows by way of example an expression level fluctuation curve obtained by plotting expression levels of the clock gene measured by the above-mentioned method at predetermined times in a day. In each diagram, the abscissa represents time, while the ordinate represents expression level.

An expression level fluctuation curve can be obtained by visual inspection from plots of expression levels. To obtain a more accurate curve, a period calculation method such as the auto-correlation method) (correlogram), the power spectrum method, the cosinor method or the periodogram method can also be used.

FIG. 1(A) illustrates by way of example a case in which the expression level was assayed as a low value (1) at 0:00 and as a highest value (h) at 12:00. Ordinary clock times such as "0:00" and "12:00" will hereinafter be called "objective clock times."

As illustrated in FIG. 1(B), on the other hand, clock times at which the expression level of the clock gene in this tested individual reaches a lowest value (1), a highest value (h) and an intermediate value (m) are represented by "T₁," "Tₕ" and "Tₘ₁ or Tₘ₂, " respectively. These T₁, Tₕ and Tₘ will be defined as "subjective clock times" in the sense that "they are clock times determined based on variations over time in the expression level of the clock gene in the tested individual." The expression level of a clock gene shows variations over time, which are inherent to each tested individual. These subjective times are, therefore, clock times inherent to each tested individual.

As shown in these diagrams, the expression level of a clock gene undergoes daily fluctuations, and shows a circadian rhythm. It is, therefore, possible to obtain information on a biorhythm of a tested individual based on variations over time in the expression level of a clock gene. Using, as a "molecular clock table," an expression level fluctuation curve which indicates variations over time in the expression level of a clock gene, a description will hereinafter be made about a method for estimating a biorhythm of a tested individual.

Now assume, for example, that, with respect to a tested individual who is known to have the expression level fluctuation curves (which will hereinafter be used synonymously with "molecular clock tables") of FIG. 1, the expression level assayed at a predetermined clock time is h. In this case, based on the expression level fluctuation curves (molecular clock tables) of FIGS. 1(A) and 1(B), the circadian rhythm of the tested individual can be estimated to be at 12:00 in terms of objective clock time and at Tₕ in terms of subjective clock time. When the activity level is 1, the circadian rhythm of the tested individual can be estimated to be at 0:00 in terms of objective clock time and at Tₗ in terms of subjective clock time. Further, when the activity level is m, the circadian rhythm can be estimated to be at 6:00 or 18:00 in terms of objective clock time and at Tₘ₁ or Tₘ₂ in terms of subjective clock time.

Next assume that with respect to the same tested individual, the expression levels obtained by conducting an assay twice, for example, at an interval of 3 hours are p and q, respectively. If q is higher than p (p < q) at that time, the circadian rhythm of the tested individual can be estimated to be between 0:00 and 12:00, in terms of objective clock time, during which the expression level is in an upward phase. In terms of subjective clock times, on the other hand, the circadian rhythm can be estimated to be between T₁ and Tₕ. If q is lower than p (q < p), on the other hand, the circadian rhythm of the tested individual can be estimated to be between 12:00 and 24:00, in terms of objective clock time, and between Tₕ and T₁, in terms of subjective clock time, during both of which the expression level is in a downward phase. In addition, the objective and subjective clock times in the circadian rhythm can be more accurately estimated by determining a coefficient of fluctuation between p and q (q/p) and correlating it with the gradient of a tangential line to the expression level fluctuation curve.

As described above, it is possible to set an optimal dosage time, activity time or meal ingestion time by finding out subjective clock times of a tested individual based on an expression level fluctuation curve (molecular clock table) which indicates variations over time of the expression level of a clock gene. As mentioned above, a biorhythm widely governs biological responses (physiological processes) such as sleep-wake cycles, body temperature, blood pressure, hormonal secretions, mental and physical activities, locomotor ability, drug sensitivity, and the like. Therefore, optimal bedtime and wake-up time, dosage time, activity time, meal ingestion time and the like can be set when such physiological processes are estimated based on subjective clock times.

By finding out objective and subjective clock times based on a molecular clock table, it is also possible to detect a shift in the biorhythm of a tested individual if such a shift arises. A specific description will hereinafter be made about a method for detecting a shift in the biorhythm of a tested individual.

An expression level fluctuation curve (molecular clock table) can be characterized in profile by its highest value (or lowest value), the observation clock time of the highest value (or lowest value), an inclination from the highest value to the lowest value (or from the lowest value to the highest value), and the like. In the present invention, this profile of the expression level fluctuation curve (molecular clock table) will be called "phase." The profile of a circadian rhythm of a tested individual as specified by an expression level fluctuation curve (molecular clock table) will also be called "phase."

A biorhythm shift can be detected based on a variation in the phase of the expression level fluctuation curve (molecular clock table).

Described specifically, the expression level fluctuation curve (molecular clock table) illustrated in FIG. 1(A) has a profile, in other words, "a phase" characterized by the lowest value 1, the highest value h, the objective clock time 0:00 of the lowest value, and the objective clock time 12:00 of the highest value.

FIG. 2 diagrammatically illustrates phase variations of expression level fluctuation curves. In FIG. 2(A), the expression level fluctuation curve represented by a dotted line is the curve (which may hereinafter be also called "the molecular clock table 1") shown in FIG. 1(A), while a solid line represents an illustrative expression level fluctuation curve (which may hereinafter be also called "the molecular clock table 2") obtained by conducting a similar assay as in FIG. 1(A) on a different assay date. In each diagram, the abscissa represents time, while the ordinate represents expression level.

The molecular clock table 1 has a phase in which the objective clock time corresponding to a lowest value (1) is 0:00 and the objective clock time corresponding to a highest value (h) is 12:00. In the molecular clock table 2, on the other hand, the objective clock time corresponding to a lowest value (1) is 6:00 and the objective clock time corresponding to a highest value (h) is 18:00, and therefore, its phase has varied. This can also be taken as variations in the subjective clock times (T₁, Tₕ) in the tested individual.

Described specifically, a shift took place in the phase of an expression level fluctuation curve of the tested individual between the time point of the preparation of the molecular clock table 1 and the time point of the preparation of the molecular clock table 2, so that the biorhythm of the tested individual at the time point of the preparation of the molecular clock table 2 was delayed by 6 hours (or advanced by 18 hours) in terms of objective clock time from the time point of the preparation of the molecular clock table 1. As described above, it is possible to detect a shift in the phase of the biorhythm of a tested individual by preparing a molecular clock table a plurality of times with respect to the same tested individual and correlating the resulting molecular clock tables with each other.

As an alternative method for detecting a shift in the phase of a biorhythm, a method such as that to be described below can be contemplated.

FIG. 2(B) is a diagram illustrating the molecular clock table 1 (also see FIG. 1) and molecular clock table 2 of FIG. 2(A) by changing the former from the dotted line to a solid line and the latter from the solid line to a dotted line.

As described above, when the activity value assayed at a predetermined clock time is m with respect to a tested individual who is known to have the expression level fluctuation curve (molecular clock table) of FIG. 1(A), the circadian rhythm of the tested individual can be estimated to be at 6:00 or 18:00 in terms of objective clock time based on the molecular clock table 1.

Now assume that with respect to the same tested individual, the activity value obtained by conducting an assay at 6:00 on a different assay date changed from m to l (see the circle dot in FIG. 2(B)). In this case, it can be estimated that the circadian rhythm of the tested individual was delayed by 6 hours (or advanced by 18 hours) in terms of objective clock time and varied to the circadian rhythm shown in the molecular clock table 2. This can also be taken as a variation in the subjective clock time (T₁) of the tested individual.

A phase shift in the biorhythm of a tested individual can also be more simply detected by correlating the activity value at a predetermined molecular clock table with a molecular clock table, which has been prepared beforehand, as described above.

As has been described above, the method according to the present invention makes it possible to estimate a biorhythm inherent to each tested individual and to detect a shift in the biorhythm by using, as a molecular clock table, an expression level fluctuation curve which indicates variations over time in the expression level of a clock gene.

Therefore, the method according to the present invention can be used for the prevention of various diseases caused by disorders (shifts) in biorhythm and also for the diagnosis and treatment of poor physical conditions such as time lag. If correlations can be revealed between the subjective times of a tested individual and various indices indicating physiological processes such as body temperature, blood temperature and hormonal secretions, these indices can be measured based on the subjective clock times, and can then be used for the diagnosis and treatment of diseases caused by disorders in the biorhythm.

In the following examples, specific assay data of expression levels of clock genes in hair follicle cells will be given.

### Examples

### (Example 1) <Expression quantification 1 of clock genes by use of hairs>

In Example 1, the quantification of expression levels was conducted with respect to clock genes in follicle cells of hairs. Twenty hairs were used in this example.

Hairs were plucked off from three human subjects (human subjects K, T, A) to obtain hair follicle cells. The collection of the hair follicle cells was conducted 9 times at 11:00, 14:00, 17:00, 20:00, 23:00, 2:00, 5:00, 8:00 and 11:00. Using a commercially-available, RNA extraction kit ("RNAeasy micro kit," Qiagen K.K.), total RNA was extracted from the hair follicle cells in accordance with an accompanying protocol. After a reverse transcription reaction ("Ribatora ace," Toyobo Co., Ltd.) was conducted in a usual way, gene expression levels were assayed with respect to Per3 gene, Nrld1 gene, Nr1d2 gene and Dbp gene by using a real-time PCR system ("Prism 7000," Applied Biosystems). The sequences of primers used for the respective genes are shown in Table 1.

**[Table 1]**

| Gene | Forward primer | SEQ ID NO | Reverse primer | SEQ ID NO |
|---|---|---|---|---|
| Per3 | CTACCTGCACCCTGAAGATCGTTCTC | 15 | CTGGAATCCAGTATGATGTAGTCTCCGTTT | 16 |
| Per2 | GCTTCCCTTGTGGAGCATGT | 17 | TTGACCAGGTAGGGCGTCAT | 18 |
| Bmal1 | CATGCAACGCAATGTCCAGGAAATTAGATA | 19 | CCTGAGAATGAGGTGTTTCAATTCATCGTC | 20 |
| Npas2 | CGGGACCAGTTCAATGTTCTCATCAAAGAG | 21 | GTCTTGCTGAATGTCACAGATTTCCGTTT | 22 |
| Nrld1 | GCTCAGTGCCATGTTCGACTTC | 23 | AAGTCTCCAAGGGCCGGTTC | 24 |
| Nrld2 | TCCAGTACAAGAAGTGCCTGAAGAATGAAA | 25 | CACGCTTAGGAATACGACCAAACCGA | 26 |
| Dbp | TCGTTGTTCTTGTACCGCCGGC | 27 | CCAGCTGATCTTGCCCTATCAAGCATTC | 28 |
| 18s rRNA | CGCCGCTAGAGGTGAAATTC | 29 | CGAACCTCCGACTTTCGTTCT | 30 |

The results are shown in FIG. 3. FIG. 3(A) shows the results on Per3 gene, FIG. 3(B) illustrates the results on Nrld1 gene, FIG. 3(C) depicts the results on Dbp gene, and FIG. 4(D) shows the results on Nrld2 gene. In each diagram, the plots indicated by rhombus dots represent the results on the human subject K, square dots represent the results on the human subject T, and triangle dots represent the results on the human subject A. In each diagram, the abscissa represents sampling clock time, while the ordinate represents gene expression level. It is to be noted that each gene expression level was subjected to an expression level correction by using 18s rRNA as an internal standard and was then indicated as a relative value while assuming that the highest value of the corresponding gene expression level in the corresponding human subject was 100.

The Per3 gene expression level in the human subject K (see the rhombus dots in FIG. 3(A)), after initiation of sampling at 11:00, decreased at 14:00 and showed a lowest value at 17:00. Subsequently, the Per3 gene expression level increased through 20:00, 23:00, 2:00 and 5:00, and showed a highest value at 8:00. Further, the Per3 gene expression level again decreased at 11:00, and showed a similar expression level as observed at the time point of the initial sampling (11:00).

The Per3 gene expression level in the human subject T (see the square dots in FIG. 3(A)), after initiation of sampling at 11:00, showed a lowest value at 14:00 and turned into a slight increase at 17:00. Subsequently, the Per3 gene expression level increased through 20:00, 23:00, 2:00 and 5:00, and a highest value was observed at 8:00. The Per3 gene expression level was similar to that in the human subject K in that it again decreased at 11:00 and showed a similar expression level as observed at the time point of the initial sampling (11:00). Further, the Per3 gene expression level in the human subject A (see the triangle dots in FIG. 3(A)) showed substantially similar variations over time as that in the human subject K.

From the foregoing, it has been indicated that the circadian rhythm of Per3 gene can be detected by assaying daily fluctuations of Per3 gene based on a real-time PCR assay that uses hair follicle cells of twenty hairs. As the detected circadian rhythm of Per3 gene showed a characteristic phase in each human subject, it has been indicated that a biorhythm inherent to each human subject can be detected by the method according to the present invention.

With respect to each of Nrld1 gene, Dbp gene and Nrld2 gene shown in FIGS. 3(B) to 3(D), it was possible to detect daily fluctuations in expression level and a circadian rhythm, and therefore, to detect a biorhythm inherent to each human subject.

Paying attention to the lowest value of the daily fluctuation on each gene, the lowest value was approximately 55% of the highest value in the case of Dbp gene (C) and was approximately 35% of the highest value in the case of each of Nrld1 gene (B) and Nrld2 gene (D), while the lowest value in the case of Per3 gene (A) was approximately 20% of the highest value, and therefore, was pronouncedly lower compared with those for the other genes. Namely, Per3 gene can be considered to be greater in the range of daily fluctuations compared with the other genes and to permit clearly detecting variations in expression level. It has, therefore, been revealed that the use of Per3 gene makes it possible to more accurately estimate a biorhythm.

### (Example 2) <Expression quantification 2 of clock genes by use of hairs>

In Example 2, the quantification of expression levels was conducted with respect to clock genes in follicle cells of hairs. A half of the hairs in Example 1, ten hairs, were used in this example.

Hairs were plucked off from one human subject (human subject B) to obtain hair follicle cells. The collection of the hair follicle cells was conducted 6 times at 12:00, 16:00, 20:00, 24:00, 4:00, and 8:00. In a similar manner as in Example 1, gene expression levels were assayed with respect to Per2 gene, Bmal1 gene, Npas2 gene and Nr1d2 gene.

The results are shown in FIG. 4. In the diagram, the plots indicated by square dots represent the results on Per2 gene, rhombus dots represent the results on Bmal1 gene, circle dots represent the results on Npas2 gene, and triangle dots represent the results on Nrld2 gene. In the diagram, the abscissa represents sampling clock time, while the ordinate represents gene expression level. It is to be noted that each gene expression level was indicated as a relative value while assuming that the expression level of 18s rRNA used as an internal standard was 1.

As shown in the diagram, it has been revealed that daily fluctuations and circadian rhythm of each gene can be detected even when the number of hairs to be used is decreased to 10.

### (Example 3) <Expression quantification 3 of clock genes by use of hairs>

In Example 3, the number of hairs to be used was decreased further to 5, and the quantification of expression levels of clock genes was conducted.

Hairs were plucked off from three human subjects (human subjects S1, S2, S3) to obtain hair follicle cells. The collection of the hair follicle cells was conducted 9 times at 12:00, 15:00, 18:00, 21:00, 24:00, 3:00, 6:00, 9:00 and 12:00. In a similar manner as in Example 1, gene expression levels of Per3 gene were assayed.

The results are shown in FIG. 5. FIG. 5(A) shows the results on the human subject S1, FIG. 5(B) illustrates the results on the human subject S2, and FIG. 5(C) depicts the results on the human subject S3. In each diagram, the abscissa represents sampling clock time, while the ordinate represents gene expression level. It is to be noted that each gene expression level was subjected to an expression level correction by using 18s rRNA as an internal standard and was then indicated as a relative value while assuming that the highest value of the corresponding expression level in the corresponding human subject was 100.

As shown in the diagrams, it has been revealed that, even when the number of hairs to be used is decreased to 5, daily fluctuations and circadian rhythm of Per3 gene can be detected and a biorhythm inherent to each human subject can be detected.

### (Example 4) <Expression quantification 1 of clock genes by use of body hairs>

In Example 4, the quantification of expression levels of clock genes in hair follicle cells was conducted by using beards. Five or three beards were used in this example.

Beards were plucked off from two human subjects (human subjects C, D) to obtain hair follicle cells. The collection of the hair follicle cells was conducted 7 times at 12:00, 16:00, 20:00, 24:00, 4:00, 8:00, and 12:00. In a similar manner as in Example 1, gene expression levels were assayed with respect to Per3 gene, Bmal1 gene, Nrld1 gene, Nrld2 gene and Dbp gene.

The results are shown in FIG. 6. FIG. 6(A) shows the results of an assay conducted by using five beards plucked off from the human subject C, and FIG. 6(B) illustrates the results of an assay conducted by using three beards plucked off from the human subject D. In each diagram, the plots indicated by squares represent the results on Per3 gene, rhombus dots represent the results on Bmal1 gene, inverted triangle dots represent the results on Nr1d1 gene, triangle dots represent the results on Nrld2 gene, and circles represent the results on Dbp gene. In each diagram, the abscissa represents sampling clock time, while the ordinate represents gene expression level. It is to be noted that each gene expression level was subjected to an expression level correction by using 18s rRNA as an internal standard and was then indicated as a relative value while assuming that the highest value of the expression level of the corresponding gene in the corresponding human subject was 1.

As shown in the diagrams, it has been revealed that, when beards are used in place of hairs, daily fluctuations and circadian rhythm of each gene can also be detected and a biorhythm inherent to each human subject can also be detected. With respect to beards, the use of three beards permitted the detection of a biorhythm.

Concerning Per3 gene, a circadian rhythm with a greater amplitude than those of the other genes was recognized as in the case of hairs.

### (Example 5) <Expression quantification 3 of clock genes by use of body hairs>

In Example 5, the quantification of expression levels of clock genes in hair follicle cells was conducted by using arm hairs (between the wrist to the elbow). Twenty arm hairs were used in this example.

Arm hairs were plucked off from one human subject (human subject E) to obtain hair follicle cells. The collection of the hair follicle cells was conducted 6 times at 18:00, 22:00, 2:00, 6:00, 10:00, and 14:00. In a similar manner as in Example 1, gene expression levels were assayed with respect to Per2 gene and Bmal1 gene.

The results are shown in FIG. 7. In the diagram, the plots indicated by square dots represent the results on Per2 gene, and rhombus dots represent the results on Bmal1 gene. In the diagram, the abscissa represents sampling clock time, while the ordinate represents gene expression level. It is to be noted that each gene expression level was subjected to an expression level correction by using 18s rRNA as an internal standard and was then indicated as a relative value while assuming that the lowest value of the expression level of the corresponding gene was 1.

As shown in the diagram, it has been revealed that, when arm hairs are used in place of hairs, daily fluctuations and circadian rhythm of each gene can be detected. With respect to arm hairs, the use of twenty arm hairs permitted the detection of a biorhythm.

### Industrial Applicability

The method according to the present invention makes it possible to estimate a biorhythm inherent to each tested individual in a simple and low-invasive manner by using, as a molecular clock table, an expression level fluctuation curve which indicates variations over time in the expression level of a clock gene in a follicle cell. It is, therefore, possible for each individual to find out a biorhythm inherent to himself or herself and to set an optimal dosage time, activity time and/or meal ingestion. The method according to the present invention can thus be used for the realization of chronotherapy, the exhibition of one's own ability, and/or dieting.

Further, the detection of a shift in the phase of a biorhythm by the method according to the present invention is useful for the prevention of various diseases caused by the biorhythm shift and also for the improvement of poor physical conditions such as time lag.

## Claims

1. A method for obtaining information on a biorhythm of an individual organism based on variations over time in an expression level of a clock gene, wherein:
(a) the clock gene is the Per3 clock gene described in Sequence ID No: 1 or a homolog thereof; and
(b) Per3 levels are measured directly in a hair follicle cell adhering to a hair root portion of a body hair plucked from the individual organism by extracting RNA from the hair follicle cell.

2. The method according to claim 1, wherein the information on a biorhythm is obtained from the expression level of at least one additional clock gene.

3. The method according to claim 2, wherein the at least one additional clock gene is selected from the following genes or homologs thereof (1) to (6):
(1) Per2 gene described in Sequence ID NO: 3 or a homolog thereof,
(2) Bmal1 gene described in Sequence ID NO: 5 or a homolog thereof,
(3) Npas2 gene described in Sequence ID NO: 7 or a homolog thereof,
(4) Nrld1 gene described in Sequence ID NO: 9 or a homolog thereof,
(5) Nr1d2 gene described in Sequence ID NO: 11 or a homolog thereof, and
(6) Dbp gene described in Sequence ID NO: 13 or a homolog thereof.

4. The method according to any one of claims 1 to 3, wherein an expression level fluctuation curve indicating the variations over time in the expression level for the or each clock gene is used as a molecular clock table for the estimation of the biorhythm.

5. The method according to claim 4, wherein a phase shift of the biorhythm of the individual organism is detected by preparing the molecular clock table for the or each clock gene a plurality of times about the individual organism and collating the resulting molecular clock tables with each other.

6. The method according to claim 4, wherein a phase shift of the biorhythm of the individual organism is detected by collating an expression level of the or each clock gene at a predetermined time with the molecular clock table.

## Patentansprüche

1. Verfahren zum Erhalten von Informationen über einen Biorhythmus eines individuellen Organismus, basierend auf zeitlichen Schwankungen eines Expressionsniveaus eines Clock-Gens, wobei:
(a) es sich bei dem Clock-Gen um das in SEQ ID NR. 1 beschriebene Per3-Clock-Gen oder ein Homolog davon handelt; und
(b) die Per3-Niveaus direkt in einer Haarfollikelzelle, die einem Haarwurzelabschnitt eines von dem individuellen Organismus herausgezupften Körperhaars anhaftet, gemessen wird, und zwar dadurch, dass RNA aus der Haarfollikelzelle extrahiert wird.

2. Verfahren nach Anspruch 1, wobei die Informationen über einen Biorhythmus von dem Expressionsniveau von mindestens einem zusätzlichen Clock-Gen erhalten werden.

3. Verfahren nach Anspruch 2, wobei das mindestens eine zusätzliche Clock-Gen aus den folgenden Genen oder Homologen davon (1) bis (6) ausgewählt ist:
(1) dem in SEQ ID NR: 3 beschriebenen Per2-Gen oder einem Homolog davon,
(2) dem in SEQ ID NR: 5 beschriebenen Bmal1-Gen oder einem Homolog davon,
(3) dem in SEQ ID NR: 7 beschriebenen Npas2-Gen oder einem Homolog davon,
(4) dem in SEQ ID NR: 9 beschriebenen Nr1d1-Gen oder einem Homolog davon,
(5) dem in SEQ ID NR: 11 beschriebenen Nr1d2-Gen oder einem Homolog davon,
(6) dem in SEQ ID NR: 13 beschriebenen Dbp-Gen oder einem Homolog davon.

4. Verfahren nach einem der Ansprüche 1 bis 3, wobei eine Expressionsniveaufluktuationskurve, die die zeitlichen Schwankungen des Expressionsniveaus des Clock-Gens bzw. jedes Clock-Gens angibt, als molekulare Uhr-Tabelle zum Abschätzen des Biorhythmus verwendet wird.

5. Verfahren nach Anspruch 4, wobei eine Phasenverschiebung des Biorhythmus des individuellen Organismus dadurch nachgewiesen wird, dass man die molekulare Uhr-Tabelle für das Clock-Gen bzw. jedes Clock-Gen mehrmals für den individuellen Organismus erstellt und die entstandenen molekularen Uhr-Tabellen miteinander kollationiert.

6. Verfahren nach Anspruch 4, wobei eine Phasenverschiebung des Biorhythmus des individuellen Organismus dadurch nachgewiesen wird, dass man ein Expressionsniveau des Uhr-Gen oder jedes Uhr-Gens zu einem vorbestimmten Zeitpunkt mit der molekularen Uhr-Tabelle kollationiert.

## Revendications

1. Procédé pour obtenir des informations sur un biorythme d'un organisme individuel basé sur des variations au cours du temps d'un niveau d'expression d'un gène d'horloge, dans lequel :
(a) le gène d'horloge est le gène d'horloge Per3 décrit dans Séquence ID n° 1 ou un homologue de celui-ci ; et
(b) les taux de Per3 sont mesurés directement dans une cellule de follicule pileux adhérant à une partie de racine pileuse d'un poil corporel prélevé à partir de l'organisme individuel par extraction d'ARN à partir de la cellule de follicule pileux.

2. Procédé selon la revendication 1, dans lequel les informations sur un biorythme est obtenu à partir du niveau d'expression d'au moins un gène d'horloge additionnel.

3. Procédé selon la revendication 2, dans lequel l'au moins un gène d'horloge additionnel est choisi parmi les gènes suivants ou homologues de ceux-ci (1) à (6) :
(1) le gène Per2 décrit dans Séquence ID n° 3 ou un homologue de celui-ci,
(2) le gène Bmall décrit dans Séquence ID n° 5 ou un homologue de celui-ci,
(3) le gène Npas2 décrit dans Séquence ID n° 7 ou un homologue de celui-ci,
(4) le gène Nr1d1 décrit dans Séquence ID n° 9 ou un homologue de celui-ci,
(5) le gène Nr1d2 décrit dans Séquence ID n° 11 ou un homologue de celui-ci, et
(6) le gène Dbp décrit dans Séquence ID n° 13 ou un homologue de celui-ci.

4. Procédé selon l'une quelconque des revendications 1 à 3, dans lequel une courbe de fluctuation de niveau d'expression indiquant les variations au cours du temps du niveau d'expression pour le ou chaque gène d'horloge est utilisé en tant que table d'horloge moléculaire pour l'estimation du biorythme.

5. Procédé selon la revendication 4, dans lequel un déphasage du biorythme de l'organisme individuel est détecté par préparation de la table d'horloge moléculaire pour le ou chaque gène d'horloge une pluralité de fois pour l'organisme individuel et le groupage des tables d'horloge moléculaire résultantes les unes avec les autres.

6. Procédé selon la revendication 4, dans lequel un déphasage du biorythme de l'organisme individuel est détecté par groupage d'un niveau d'expression du ou de chaque gène d'horloge à un temps prédéterminé avec la table d'horloge moléculaire.


### SEQUENCE LISTING

<110> Sony Corporation
<120> Acquisition of biological rhythm information from hair cells
<130> 0690632201
<160> 30
<170> PatentIn version 3.1
<210> 1
   <211> 6203
   <212> DNA
   <213> Homo sapiens
<220>
   <221> CDS
   <222> (176)..(3781)
   <223>
<400> 1
ggaaaagctc ctcggagatg agcgtgaccc cctggctcgt ggtggccgcc tgttctcact    60
   aacgccatgg cggggaccgg agtgagaaac cggtgtctgt cactgactgc aaagtgagcg   120
   agaagcaggc tgcgggccgt cccagcacga cgtggagccc cgcggagacc tcgag atg    178
   ccc cgc ggg gaa gct cct ggc ccc ggg aga cgg ggg gct aag gac gag     226
   gcc ctg ggc gaa gaa tcg ggg gag cgg tgg agc ccc gag ttc cat ctg     274
   cag agg aaa ttg gcg gac agc agc cac agt gaa cag caa gat cga aac     322
   aga gtt tct gaa gaa ctt atc atg gtt gtc caa gaa atg aaa aaa tac     370
   ttc ccc tcg gag aga cgc aat aaa cca agc act cta gat gcc ctc aac     418
   tat gct ctc cgc tgt gtc cac agc gtt caa gca aac agt gag ttt ttc     466
   cag att ctc agt cag aat gga gca cct cag gca gat gtg agc atg tac     514
   agt ctt gag gag ctg gcc act atc gct tca gaa cac act tcc aaa aac      562
   aca gat acc ttt gtg gca gta ttt tca ttt ctg tct gga agg tta gtg      610
   cac att tct gaa cag gct gct ttg atc ctg aat cgt aag aaa gat gtc      658
   ctg gcg tct tct cac ttt gtt gac ctg ctt gca cct caa gac atg agg      706
   gta ttc tac gcg cac act gcc aga gct cag ctt cct ttc tgg aac aac      754
   tgg acc caa aga gct gca cgg tat gaa tgt gct ccg gtg aaa cct ttt      802
   ttc tgc agg atc cgt gga ggt gaa gac aga aag caa gag aag tgt cac      850
   tcc cca ttc cgg atc atc ccc tat ctg att cat gta cat cac cct gcc      898
   cag cca gaa ttg gaa tcg gaa cct tgc tgt ctc act gtg gtt gaa aag      946
   att cac tct ggt tat gaa gct cct cgg atc cca gtg aat aaa aga atc      994
   ttc acc acc aca cac acc cca ggg tgt gtt ttt ctt gaa gta gat gaa      1042
   aaa gca gtg cct ttg ctg ggt tac cta cct cag gac ctg att gga aca      1090
   tcg atc cta agc tac ctg cac cct gaa gat cgt tct ctg atg gtt gcc      1138
   ata cac caa aaa gtt ttg aag tat gca ggg cat cct ccc ttt gaa cat      1186
   tct ccc att cga ttt tgt act caa aac gga gac tac atc ata ctg gat      1234
   tcc agt tgg tcc agc ttt gtg aat ccc tgg agc cgg aag att tct ttc 1282
   atc att ggt cgg cat aaa gtt cga acg agc cca cta aat gag gat gtt 1330
   ttt gct acc aaa att aaa aag atg aac gat aat gac aaa gac ata aca 1378
   gaa tta caa gaa caa att tac aaa ctt ctc tta cag cca gtt cac gtg 1426
   agc gtg tcc agc ggc tac ggg agc ctg ggg agc agc ggg tcg cag gag 1474
   cag ctt gtc agc atc gcc tcc tcc agt gag gcc agt ggg cac cgt gtg 1522
   gag gag acg aag gcg gag cag atg acc ttg cag cag gtc tat gcc agt 1570
   gtg aac aaa att aaa aat ctg ggt cag cag ctc tac att gag tca atg 1618
   acc aaa tca tca ttc aag cca gtg acg ggg aca cgc aca gaa ccg aat 1666
   ggt ggt ggt gaa tgt aag acc ttt act tcc ttc cac caa aca ctg aaa 1714
   aac aat agt gtg tac act gag ccc tgt gag gat ttg agg aac gat gag 1762
   cac agc cca tcc tat caa cag atc aac tgt atc gac agt gtc atc aga 1810
   tac ctg aag agc tac aac att cca gct ttg aaa aga aag tgt atc tcc 1858
   tgt aca aat aca act tct tcc tcc tca gaa gaa gac aaa cag aac cac 1906
   aag gca gat gat gtc caa gcc tta caa gct ggt ttg caa atc cca gcc 1954
   ata cct aaa tca gaa atg cca aca aat gga cgg tcc ata gac aca gga      2002
   gga gga gct cca cag atc ctg tcc acg gcg atg ctg agc ttg ggg tcg      2050
   ggc ata agc caa tgc ggt tac agc agc acc att gtc cat gtc cca ccc      2098
   cca gag aca gcc agg gat gct acc ctc ttc tgt gag ccc tgg acc ctg      2146
   aac atg cag cca gcc cct ttg acc tcg gaa gaa ttt aaa cac gtg ggg      2194
   ctc aca gcg gct gtt ctg tca gcg cac acc cag aag gaa gag cag aat      2242
   tat gtt gat aaa ttc cga gaa aag atc ctg tca tca ccc tac agc tcc      2290
   tat ctt cag caa gaa agc agg agc aaa gct aaa tat tca tat ttt caa      2338
   gga gat tct act tcc aag cag acg cgg tcg gcc ggc tgc agg aaa ggg      2386
   aag cac aag cgg aag aag ctg ccg gag ccg cca gac agc agc agc tcg      2434
   aac acc ggc tct ggt ccc cgc agg gga gcg cat cag aac gca cag ccc      2482
   tgc tgc ccc tcc gcg gcc tcc tct ccg cac acc tcg agc ccg acc ttc      2530
   cca cct gcc gcc atg gtg ccc agc cag gcc cct tac ctc gtc cca gct      2578
   ttt ccc ctc cca gcc gcg acc tca ccc gga aga gaa tac gca gcc ccc      2626
   gga act gca ccg gaa ggc ctg cat ggg ctg ccc ttg tcc gag ggc ttg      2674
   cag cct tac cca gct ttc cct ttt cct tac ttg gat act ttt atg acc      2722
   gtt ttc ctg cct gac ccc cct gtc tgt cct ctg ttg tcg cca tcg ttt      2770
   ttg cca tgt cca ttc ctg ggg gcg aca gcc tct tct gcg ata tca ccc      2818
   tca atg tcg tca gca atg agt cca act ctg gac cca ccc cct tca gtc      2866
   acc agc caa agg aga gag gag gaa aag tgg gag gca caa agc gag ggg      2914
   cac ccg ttc att act tcg aga agc agc tca ccc ttg cag tta aac tta      2962
   ctt cag gaa gag atg ccc aga ccc tct gaa tct cca gat cag atg aga      3010
   agg aac acg tgc cca caa act gag tat tgt gtt aca ggc aac aat ggc      3058
   agt gag agc agt cct gct act acc ggt gca ctg tcc acg ggg tca cct      3106
   ccc agg gag aat cca tcc cat cct act gcc agc gct ctg tcc aca gga      3154
   tcg cct ccc atg aag aat cca  tcc cat cct act gcc  agc gct ctg tcc    3202
   aca  gga tcg cct ccc atg  aag aat cca tcc cat  cct act gcc agc      3247
   aca  ctg tcc atg gga ttg  cct ccc agc agg act  cca tcc cat cct      3292
   act  gcc act gtt ctg tcc  acg ggg tca cct ccc  agc gaa tcc cca      3337
   tcc  aga act ggt tca gca  gca tca gga agc agc  gac agc agt ata      3382
   tac ctt act agt agt gtt tat tct tct aaa atc tcc caa aat ggg      3427
   cag caa tct cag gac gta cag aaa aaa gaa aca ttt cct aat gtc      3472
   gcc gaa gag ccc atc tgg aga atg ata cgg cag aca cct gag cgc      3517
   att ctc atg aca tac cag gta cct gag agg gtt aaa gaa gtt gta      3562
   cta aaa gaa gac ctg gaa aag cta gaa agt atg agg cag cag cag      3607
   ccc cag ttt tct cat ggg caa aag gag gag ctg gct aag gtg tat      3652
   aat tgg att caa agc cag act gtc act caa gaa atc gac att caa      3697
   gcc tgt gtc act tgt gaa aat gaa gat tca gct gat ggt gcg gcc      3742
   aca tcc tgt ggt cag gtt ctg gta gaa gac agc tgt tga gtgactgtga   3791
   ggatgaacct tcataccctt tccaagacgt gttacacaga cagacctttt taagtcctgg   3851
   acttttaaat gaccatgaag ttatcattga atgttaagat tttttcttct tgatttttta   3911
   atacacgtaa tctttttgaa gcagacattg tatacagaat cttacttctc tttgttcctg   3971
   atatattaaa atggccagtt aggctctttt tgtagttgaa ttgtcttcta aagagattgg   4031
   atggcctcta aagaggtatg tgtatcttta tttcagatgt cacccagagt aaattataat   4091
   tagaagtata gctagaatga gccccaaacc ttagcctcat ttattttgtt ctgttacata   4151
   agtcattttc cccttagagt gcttgaagaa atgccaccta caggttgtgt acttttcata   4211
   atggtttcca tgaatgtagt acgttcatac aggcttcatt caacctggcg ttcccctcca   4271
   taattaagat gaaacattcc ggttttctca caacacatta gcacatactg tccattagca   4331
   tatctgggat aaccaggttt tgggggttga gttttggcct tcatccttgt agatcccttt   4391
   cctattgatt tcccaccttc cagtgaaatt ctgaaagtct tatcttaaaa atcgatccgc   4451
   ttaccatggg cctattcttg taagtttcag ttagcatttg catgtgtaat attaaaatga   4511
   aagagcttct tacccagtgc tgttgccctt ttgagtattt ttgtttttaa aataatgatt   4571
   gtaaaatgtt ttacaagtaa tgtaaaagct agtatcattc ttacatactt ctgtgtttaa   4631
   attttcattc ttaccaaaac agttaactct ttctttccaa tcaatttata caaaagaggt   4691
   cgctccagcc ctaccacagg tctgactggc actgcctttt gtttgccctt gaacagggca   4751
   gtgttgtggg gactgcaaaa gagaaaacgt ccaggcgagc ccagttgtcc tcgcccacag   4811
   ggtcctgcag gctccatcag tcaccgcttt ctatggcgtt tgtagttgtg tcttttaaga   4871
   agtgagtgtg attgtttact tgataaatca gctcactctc tggtgctttt tagagaagtc   4931
   cctgattcct tcttaaactt ggaatgatag atgaaattca cacccctgca gatcagaaaa   4991
   aacaaataga agaaaatgag ggttacagta acctgttgtc tttatataac ttgcaacaaa   5051
   ctaatttatt tttttttcct ttttttgttt ttggtttttt atggtttttt aaggaaaata   5111
   cttttctcct ttgaagtttt acagcttttt gtaaatgcgt cctgataatg attaggaaaa   5171
   tcgacctttt catccatgat gaccatcctc atagctcaga tctcctttca aagtagtggc   5231
   tttctggatg gtaattccat cttaaggtgt cagaactatt ttcaaatgct gcctttgaca   5291
   gttcttggaa ttttctgata ttaagcagtt ccatgcaaat attcgtgttt tataaatagc   5351
   tctcatagtc tgctccatct tgatagttaa gtgatttctg aagcgtttgt gtgtgtgttg   5411
   atcaggttgt gtgatatttt tgcttgatag agaatcaaat ttgaaacaat taaccagcca   5471
   gtagattgtc tgtcagtgac cttctgtagt aataaagttt ttgccactgt aaataaaaac   5531
   agtatccgta gctatcagga tcattgcgca ctcatatatg ctaagccttc tgttctctaa   5591
   tagaagcctt tcttttccat tgtttctgga tatttgtatt atccaaatgt gcttatttct   5651
   ttgccttagc acacgtttta tggagtactt gttatactag gtttgatttg aaactggtgc   5711
   ttgtcgcaga actgtcagag catgaggagc gctcctcctg tgggtggacg cattcacgca   5771
   ctcccaggtt gcacctgctg ctggcggtga gcagggggtt cagcagcttg accgatgccc   5831
   cccgaggggg ctctccccag cttaaacttt gttgtttaaa tttgttaact ttttatatta   5891
   atgactattg aaagtggtaa taaaaattta tattataggc ttcaatgttt tcatgaatgt   5951
   tacccaaaaa gctgtgtttt ctttggtcag aggtcaaaat ttatgaaaaa caaaatgctg   6011
   tatgaatgga aatcattttg caattgagtg acacttcatt gtaattcaca gtgtaaattt   6071
   aatccaaact gaaattttgt ttcaactgaa tttgtaatta actctgaatt tgtttttaat   6131
   cattagtaat atttcagttg ggtatctttt taagtaaaaa caacaaataa actctgtaca   6191
   tgtaaaacgt ga                                                       6203
<210> 2
   <211> 1201
   <212> PRT
   <213> Homo sapiens
<400> 2
<210> 3
   <211> 6342
   <212> DNA
   <213> Homo sapiens
<220>
   <221> CDS
   <222> (238) .. (4005)
   <223>
<400> 3
   agcgccggct cgggcagcgg aggcgccgcc ggaagttcct tgggctgctg gactcctcgg      60
   cttgaaacgg cgccggcgtg ggggcgtgtg cccttggccc tgtcccaggt ggagagtggt     120
   cgagccgcgc gcagggtgcg ctcgtttgaa ctgcggtgac accgagggtt ggggactcga     180
   acccccgctt cgcagctcag gagcctgagg tccgaaagct tcgttccaga gcccagc        237
   atg aat gga tac gcg gaa ttt ccg ccc agc ccc agt aac ccc acc aag       285
   Met Asn Gly Tyr Ala Glu Phe Pro Pro Ser Pro Ser Asn Pro Thr Lys
   1               5                   10                  15
   gag ccc gtg gag ccc cag ccc agc cag gtc cca ctg cag gaa gat gtg       333
   Glu Pro Val Glu Pro Gln Pro Ser Gln Val Pro Leu Gln Glu Asp Val
               20                  25                  30
   gac atg agc agt ggc tcc agt gga cat gag acc aac gaa aac tgc tcc       381
   Asp Met Ser Ser Gly Ser Ser Gly His Glu Thr Asn Glu Asn Cys Ser
           35                  40                  45
   acg ggg cgg gac tcg cag ggc agt gac tgt gac gac agt ggg aag gag       429
   Thr Gly Arg Asp Ser Gln Gly Ser Asp Cys Asp Asp Ser Gly Lys Glu
       50                  55                  60
   ctg ggg atg ctg gtg gag cca ccg gat gcc cgc cag agt cca gat acc       477
   Leu Gly Met Leu Val Glu Pro Pro Asp Ala Arg Gln Ser Pro Asp Thr
   65                  70                  75                  80
   ttt agc ctg atg atg gca aaa tct gaa cac aac cca tct aca agt ggc       525
   Phe Ser Leu Met Met Ala Lys Ser Glu His Asn Pro Ser Thr Ser Gly
                   85                  90                  95
   tgc agt agc gac cag tct tcg aaa gtg gac aca cac aaa gaa ctg ata       573
   Cys Ser Ser Asp Gln Ser Ser Lys Val Asp Thr His Lys Glu Leu Ile
               100                 105                 110
   aaa aca cta aag gag ctg aag gtc cac ctc cct gca gac aag aag gcc       621
   Lys Thr Leu Lys Glu Leu Lys Val His Leu Pro Ala Asp Lys Lys Ala
           115                 120                 125
   aag ggc aag gcc agt acg ctg gcc acc ttg aag tac gcc ctc agg agc      669
   Lys Gly Lys Ala Ser Thr Leu Ala Thr Leu Lys Tyr Ala Leu Arg Ser
       130                 135                 140
   gtg aag cag gtg aaa gcc aat gaa gag tat tac cag ctg ctg atg tcc      717
   Val Lys Gln Val Lys Ala Asn Glu Glu Tyr Tyr Gln Leu Leu Met Ser
   145                 150                 155                 160
   agc gag ggt cac ccc tgt gga gca gac gtg ccc tcc tac acc gtg gag      765
   Ser Glu Gly His Pro Cys Gly Ala Asp Val Pro Ser Tyr Thr Val Glu
                   165                 170                 175
   gag atg gag agc gtt acc tct gag cac att gtg aag aat gcc gat atg      813
   Glu Met Glu Ser Val Thr Ser Glu His Ile Val Lys Asn Ala Asp Met
               180                 185                 190
   ttt gcg gtg gcc gtg tcc ctg gtg tct ggg aag atc ctg tac atc tct      861
   Phe Ala Val Ala Val Ser Leu Val Ser Gly Lys Ile Leu Tyr Ile Ser
           195                 200                 205
   gac cag gtt gca tcc ata ttt cac tgt aaa aga gat gcc ttc agc gat      909
   Asp Gln Val Ala Ser Ile Phe His Cys Lys Arg Asp Ala Phe Ser Asp
       210                 215                 220
   gcc aag ttt gtg gag ttc ctg gcg cct cac gat gtg ggc gtg ttc cac      957
   Ala Lys Phe Val Glu Phe Leu Ala Pro His Asp Val Gly Val Phe His
   225                 230                 235                 240
   agt ttc acc tcc ccg tac aag ctt ccc ttg tgg agc atg tgc agt gga     1005
   Ser Phe Thr Ser Pro Tyr Lys Leu Pro Leu Trp Ser Met Cys Ser Gly
                   245                 250                 255
   gca gat tct ttt act caa gaa tgc atg gag gag aaa tct ttc ttt tgc     1053
   Ala Asp Ser Phe Thr Gln Glu Cys Met Glu Glu Lys Ser Phe Phe Cys
               260                 265                 270
   cgt gtc agt gtc cgg aaa agc cac gag aat gaa atc cgc tac cac ccc     1101
   Arg Val Ser Val Arg Lys Ser His Glu Asn Glu Ile Arg Tyr His Pro
           275                 280                 285
   ttc cgc atg acg ccc tac ctg gtc aag gtg cgg gac caa caa ggt gct     1149
   Phe Arg Met Thr Pro Tyr Leu Val Lys Val Arg Asp Gln Gln Gly Ala
       290                 295                 300
   gag agt cag ctt tgc tgc ctt ctg ctg gca gag aga gtg cac tct ggt     1197
   Glu Ser Gln Leu Cys Cys Leu Leu Leu Ala Glu Arg Val His Ser Gly
   305                 310                 315                 320
   tat gaa gcc cct aga att cct cct gaa aag aga att ttt aca acc acc     1245
   Tyr Glu Ala Pro Arg Ile Pro Pro Glu Lys Arg Ile Phe Thr Thr Thr
                   325                 330                 335
   cat aca cca aat tgt ttg ttc cag gat gtg gat gaa agg gcg gtc cct     1293
   His Thr Pro Asn Cys Leu Phe Gln Asp Val Asp Glu Arg Ala Val Pro
               340                 345                 350
   ctc ctg ggc tac cta cct cag gac ctg att gaa acc cca gtg ctc gtg     1341
   Leu Leu Gly Tyr Leu Pro Gln Asp Leu Ile Glu Thr Pro Val Leu Val
           355                 360                 365
   cag ctc cac cct agt gac agg ccc ttg atg ctg gcc atc cac aaa aag      1389
   Gln Leu His Pro Ser Asp Arg Pro Leu Met Leu Ala Ile His Lys Lys
       370                 375                 380
   atc ctg cag tca ggc ggg cag cct ttc gac tat tct ccc att cgg ttt      1437
   Ile Leu Gln Ser Gly Gly Gln Pro Phe Asp Tyr Ser Pro Ile Arg Phe
   385                 390                 395                 400
   cgc gcc cgg aac gga gag tac atc acg ttg gac acc agc tgg tcc agc      1485
   Arg Ala Arg Asn Gly Glu Tyr Ile Thr Leu Asp Thr Ser Trp Ser Ser
                   405                 410                 415
   ttc atc aac cca tgg agc agg aaa atc tcc ttc atc att ggg agg cac      1533
   Phe Ile Asn Pro Trp Ser Arg Lys Ile Ser Phe Ile Ile Gly Arg His
               420                 425                 430
   aaa gtc agg gtg ggc cct ttg aat gag gac gtg ttt gca gcc cac ccc      1581
   Lys Val Arg Val Gly Pro Leu Asn Glu Asp Val Phe Ala Ala His Pro
           435                 440                 445
   tgc aca gag gag aag gcc ctg cac ccc agc att cag gag ctc aca gag      1629
   Cys Thr Glu Glu Lys Ala Leu His Pro Ser Ile Gln Glu Leu Thr Glu
       450                 455                 460
   cag atc cac cgg ctc ctg ctg cag ccc gtc ccc cac agc ggc tcc agt      1677
   Gln Ile His Arg Leu Leu Leu Gln Pro Val Pro His Ser Gly Ser Ser
   465                 470                 475                 480
   ggc tac ggg agt ctg ggc agc aac ggg tcc cac gag cac ctt atg agc      1725
   Gly Tyr Gly Ser Leu Gly Ser Asn Gly Ser His Glu His Leu Met Ser
                   485                 490                 495
   cag acc tcc tcc agc gac agc aac ggc cat gag gac tca cgc cgg agg      1773
   Gln Thr Ser Ser Ser Asp Ser Asn Gly His Glu Asp Ser Arg Arg Arg
               500                 505                 510
   aga gcc gaa att tgt aaa aat ggt aac aag acc aaa aat aga agt cat      1821
   Arg Ala Glu Ile Cys Lys Asn Gly Asn Lys Thr Lys Asn Arg Ser His
           515                 520                 525
   tat tct cat gaa tct gga gaa caa aag aaa aaa tcc gtt aca gaa atg      1869
   Tyr Ser His Glu Ser Gly Glu Gln Lys Lys Lys Ser Val Thr Glu Met
       530                 535                 540
   caa act aat ccc cca gct gag aag aaa gct gtc cct gcc atg gaa aag      1917
   Gln Thr Asn Pro Pro Ala Glu Lys Lys Ala Val Pro Ala Met Glu Lys
   545                 550                 555                 560
   gac agc ctg ggg gtc agc ttc ccc gag gag ttg gcc tgc aag aac cag      1965
   Asp Ser Leu Gly Val Ser Phe Pro Glu Glu Leu Ala Cys Lys Asn Gln
                   565                 570                 575
   ccc acc tgc tcc tac cag cag atc agc tgc ttg gac agc gtc atc agg      2013
   Pro Thr Cys Ser Tyr Gln Gln Ile Ser Cys Leu Asp Ser Val Ile Arg
               580                 585                 590
   tac ttg gag agc tgc aat gag gct gcc acc ctg aag agg aaa tgc gag      2061
   Tyr Leu Glu Ser Cys Asn Glu Ala Ala Thr Leu Lys Arg Lys Cys Glu
           595                 600                 605
   ttc cca gca aac gtc cca gcg cta agg tcc agt gat aag cgg aag gcc      2109
   Phe Pro Ala Asn Val Pro Ala Leu Arg Ser Ser Asp Lys Arg Lys Ala
       610                 615                 620
   aca gtc agc cca ggg cca cac gct gga gag gca gag ccg ccc tcc agg      2157
   Thr Val Ser Pro Gly Pro His Ala Gly Glu Ala Glu Pro Pro Ser Arg
   625                 630                 635                 640
   gtg aac agc cgc acg gga gta ggt acg cac ctg acc tcg ctg gca ctg      2205
   Val Asn Ser Arg Thr Gly Val Gly Thr His Leu Thr Ser Leu Ala Leu
                   645                 650                 655
   ccg ggc aag gca gag agt gtg gcg tcg ctc acc agc cag tgc agc tac      2253
   Pro Gly Lys Ala Glu Ser Val Ala Ser Leu Thr Ser Gln Cys Ser Tyr
               660                 665                 670
   agc agc acc atc gtc cat gtg gga gac aag aag ccg cag ccg gag tta      2301
   Ser Ser Thr Ile Val His Val Gly Asp Lys Lys Pro Gln Pro Glu Leu
           675                 680                 685
   gag atg gtg gaa gat gct gcg agt ggg cca gaa tcc ctg gac tgc ctg      2349
   Glu Met Val Glu Asp Ala Ala Ser Gly Pro Glu Ser Leu Asp Cys Leu
       690                 695                 700
   gcg ggc cct gcc ctg gcc tgt ggt ctc agc caa gag aag gag ccc ttc      2397
   Ala Gly Pro Ala Leu Ala Cys Gly Leu Ser Gln Glu Lys Glu Pro Phe
   705                 710                 715                 720
   aag aag ctg ggc ctc acc aag gag gta ctc gct gca cac aca cag aag      2445
   Lys Lys Leu Gly Leu Thr Lys Glu Val Leu Ala Ala His Thr Gln Lys
                   725                 730                 735
   gag gag cag agc ttc ctg cag aag ttc aaa gaa ata aga aaa ctc agc      2493
   Glu Glu Gln Ser Phe Leu Gln Lys Phe Lys Glu Ile Arg Lys Leu Ser
               740                 745                 750
   att ttc cag tcc cac tgc cat tac tac ttg caa gaa aga tcc aag ggg      2541
   Ile Phe Gln Ser His Cys His Tyr Tyr Leu Gln Glu Arg Ser Lys Gly
           755                 760                 765
   cag cca agt gaa cga act gcc cct gga cta aga aat act tcc gga ata      2589
   Gln Pro Ser Glu Arg Thr Ala Pro Gly Leu Arg Asn Thr Ser Gly Ile
       770                 775                 780
   gat tca cct tgg aaa aaa aca gga aag aac aga aaa ttg aag tcc aag      2637
   Asp Ser Pro Trp Lys Lys Thr Gly Lys Asn Arg Lys Leu Lys Ser Lys
   785                 790                 795                 800
   cgg gtc aaa cct cga gac tca tct gag agc acc gga tct ggg ggg ccc      2685
   Arg Val Lys Pro Arg Asp Ser Ser Glu Ser Thr Gly Ser Gly Gly Pro
                   805                 810                 815
   gtg tcc gcc cgg ccc ccg ctg gtg ggc ttg aac gcc aca gcc tgg tca      2733
   Val Ser Ala Arg Pro Pro Leu Val Gly Leu Asn Ala Thr Ala Trp Ser
               820                 825                 830
   ccc tca gac acg tcc cag tcc agc tgc cca gcc gtg ccc ttt ccc gcc      2781
   Pro Ser Asp Thr Ser Gln Ser Ser Cys Pro Ala Val Pro Phe Pro Ala
           835                 840                 845
   cca gtg cca gca gct tat tca ctg ccc gtg ttt cca gcg cca ggg act       2829
   Pro Val Pro Ala Ala Tyr Ser Leu Pro Val Phe Pro Ala Pro Gly Thr
       850                 855                 860
   gtg gca gca ccc ccg gca cct ccc cac gcc agc ttc aca gtg cct gct       2877
   Val Ala Ala Pro Pro Ala Pro Pro His Ala Ser Phe Thr Val Pro Ala
   865                 870                 875                 880
   gtg ccc gtg gac ctc cag cac cag ttt gca gtc cag ccc cca cct ttc       2925
   Val Pro Val Asp Leu Gln His Gln Phe Ala Val Gln Pro Pro Pro Phe
               885                 890                 895
   cct gcc cct ttg gcg cct gtc atg gca ttc atg cta ccc agt tat tcc       2973
   Pro Ala Pro Leu Ala Pro Val Met Ala Phe Met Leu Pro Ser Tyr Ser
           900                 905                 910
   ttc ccc tcg ggg acc cca aac ctg ccc cag gcc ttc ttc ccc agc cag       3021
   Phe Pro Ser Gly Thr Pro Asn Leu Pro Gln Ala Phe Phe Pro Ser Gln
       915                 920                 925
   cct cag ttt ccg agc cac ccc aca ctc aca tcc gag atg gcc tct gcc       3069
   Pro Gln Phe Pro Ser His Pro Thr Leu Thr Ser Glu Met Ala Ser Ala
       930                 935                 940
   tca cag cct gag ttc ccc agc cgg acc tcg atc ccc aga cag cca tgt       3117
   Ser Gln Pro Glu Phe Pro Ser Arg Thr Ser Ile Pro Arg Gln Pro Cys
   945                 950                 955                 960
   gct tgt cca gcc acc cgg gcc acc cca cca tcg gcc atg ggt agg gcc       3165
   Ala Cys Pro Ala Thr Arg Ala Thr Pro Pro Ser Ala Met Gly Arg Ala
               965                 970                 975
   tcc cca ccg ctc ttt cag tcc cgc agc agc tcg ccc ctg cag ctc aac       3213
   Ser Pro Pro Leu Phe Gln Ser Arg Ser Ser Ser Pro Leu Gln Leu Asn
           980                 985                 990
   ctg ctg cag ctg gag gaa gcc cct  gag ggt ggc act gga  gcc atg ggg     3261
   Leu Leu Gln Leu Glu Glu Ala Pro  Glu Gly Gly Thr Gly  Ala Met Gly
       995                 1000                1005
   acc aca  ggg gcc aca gag aca  gca gct gta ggg gcg  gac tgc aaa        3306
   Thr Thr  Gly Ala Thr Glu Thr  Ala Ala Val Gly Ala  Asp Cys Lys
       1010                1015                1020
   cct ggc  act tct cgg gac cag  cag ccg aag gcg cct  ctg acc cgt        3351
   Pro Gly  Thr Ser Arg Asp Gln  Gln Pro Lys Ala Pro  Leu Thr Arg
       1025                1030                1035
   gat gaa  ccc tca gac aca cag  aac agt gac gcc ctt  tcc acg tca        3396
   Asp Glu  Pro Ser Asp Thr Gln  Asn Ser Asp Ala Leu  Ser Thr Ser
       1040                1045                1050
   agc ggc  ctc cta aac ctc ctg  ctg aat gag gac ctc  tgc tca gcc        3441
   Ser Gly  Leu Leu Asn Leu Leu  Leu Asn Glu Asp Leu  Cys Ser Ala
       1055                1060                1065
   tcg ggc  tct gct gct tcg gag  tct ctg ggc tcc ggc  tca ctg ggc        3486
   Ser Gly  Ser Ala Ala Ser Glu  Ser Leu Gly Ser Gly  Ser Leu Gly
       1070                1075                1080
   tgc gac  gcc tcc ccg agt ggg  gca ggc agt agt gac  aca agt cat      3531
   Cys Asp  Ala Ser Pro Ser Gly  Ala Gly Ser Ser Asp  Thr Ser His
       1085                 1090                 1095
   acc agc  aaa tat ttt gga agc  att gac tcc tca gag  aat aat cac      3576
   Thr Ser  Lys Tyr Phe Gly Ser  Ile Asp Ser Ser Glu  Asn Asn His
       1100                 1105                 1110
   aaa gca  aaa atg aac act ggt  atg gaa gaa agt gag  cat ttc att      3621
   Lys Ala  Lys Met Asn Thr Gly  Met Glu Glu Ser Glu  His Phe Ile
       1115                 1120                 1125
   aag tgc  gtc ctg cag gat ccc  atc tgg ctg ctg atg  gca gat gcg      3666
   Lys Cys  Val Leu Gln Asp Pro  Ile Trp Leu Leu Met  Ala Asp Ala
       1130                 1135                 1140
   gac agc  agc gtc atg atg acg  tac cag ctg cct tcc  cga aat tta      3711
   Asp Ser  Ser Val Met Met Thr  Tyr Gln Leu Pro Ser  Arg Asn Leu
       1145                 1150                 1155
   gaa gcg  gtt ttg aag gag gac  aga gag aag ctg aag  ctc cta cag      3756
   Glu Ala  Val Leu Lys Glu Asp  Arg Glu Lys Leu Lys  Leu Leu Gln
       1160                 1165                 1170
   aaa ctc  cag ccc agg ttc acg  gag agt cag aag cag  gag ctg cgc      3801
   Lys Leu  Gln Pro Arg Phe Thr  Glu Ser Gln Lys Gln  Glu Leu Arg
       1175                 1180                 1185
   gag gtc  cac cag tgg atg cag  acg ggc ggc ctg ccc  gca gcc atc      3846
   Glu Val  His Gln Trp Met Gln  Thr Gly Gly Leu Pro  Ala Ala Ile
       1190                 1195                 1200
   gac gtg  gca gaa tgt gtt tac  tgt gaa aac aag gaa  aaa ggt aat      3891
   Asp Val  Ala Glu Cys Val Tyr  Cys Glu Asn Lys Glu  Lys Gly Asn
       1205                 1210                 1215
   att tgc  ata cca tat gag gaa  gat att cct tct ctg  gga ctc agc      3936
   Ile Cys  Ile Pro Tyr Glu Glu  Asp Ile Pro Ser Leu  Gly Leu Ser
       1220                 1225                 1230
   gaa gtg  tcg gac acc aaa gaa  gac gaa aat gga tcc  ccc ttg aat      3981
   Glu Val  Ser Asp Thr Lys Glu  Asp Glu Asn Gly Ser  Pro Leu Asn
       1235                 1240                 1245
   cac agg  atc gaa gag cag acg  taa cccctgcccc acctcagccc             4025
   His Arg  Ile Glu Glu Gln Thr
       1250                 1255
   ggcagccagc gaggtacacc aggtggtgct tggaagagat gaaagatctt catggctgtt   4085
   tccactgaaa tggacacata tgctcatgtt gctttttttg ttttagaaaa aaaaacaaca   4145
   tagttttctg aaggggcgac ttaaaactgt ggagagtggg gagagttcgg aaagaaatat   4205
   gtttttatat ataaaatata tatgtggagt tttgtgggat ggggaagaga ttttagttgt   4265
   tatttaactt gagaaagact aagcgcctct tagtgtcagg gaagttgcct cagtgctccc   4325
   agaagtcctg tgactgtgac gagacctctg tctgctgcac cagctgggga ctctggcttc   4385
   cagagctttc ccagggtgtt tggatcagat caaattttgt cctctcttgg ggactgcttt 4445
   ttatctgaat tatcatttag tcaaggtaga gtgttttttt atacatacca aatggagata 4505
   gcagcctctc ctagttttat ttcaaaacgt ttcacattaa atggtgtgaa gcgttgtttg 4565
   gcaaaccaac agctttggct tctggtgtgg tcaatatttc agtctgacat aggttttgtt 4625
   tgtagtgaac aaagttgaaa catttgctct ggactaaaga agcctagtgg tttgtgtggc 4685
   caactccatc ggatgaatgc acacgcagac agaccctctg tatatttctg cattattctt 4745
   gtctcctttt cagaccatga tggccaatat ggagattaaa atatgtcatc agtcatctct 4805
   ttatggtgac ttccctttgc aaaccaggct gtgaccaaca catgtgagac ccagtcctgt 4865
   ttggttttct tccgttggaa ccacccagac atctgcttcc acccagccaa gcccacatca 4925
   catctcctgg ccgagagcag ccactgccac tcagtctgac agcttgcgac tgcatctgta 4985
   ttttcagggg tgcagtgagc tcacctctcc cactgcaccc tgggttgggt gcacagccct 5045
   cattcttttc atgagcccga cctctctcgg agcagcttca ggcctctgcc agtgtcccca 5105
   gcacttttag gtcatttgga cacttgggga aaagtgaggc cagtctgccc ggctttttac 5165
   aaaacctcat gttgcattgt atattccaaa gatggttcag aaaatttaat attggtccct 5225
   ggtggaaatt caaagttatc actgaagaac agttgactta aaattggacc aagactatga 5285
   ggcttaaaag ggaccagggt tttctttttt tttttttttt tttttttttt ttagatggag 5345
   tttctttttg cccaggctgg agtgcagtgg cgccatcttg gctcactgca acctctgcct 5405
   cccaggttca agcgattctc ctgcctcagc ctcctgagta gctgggacca caggcgactg 5465
   ccaccacacc cagctaattt tttgtatttt tagtagagac agggtttcac catgttggcc 5525
   aggctggtct cgaactcctg acctcaagcg atccacccac ctcggcctcc caaagtgctg 5585
   ggattacagg cgtgagccac cacgcccaac tgggaccagg gttttctgtt ttttgatgga 5645
   ggtgaaatct ctttgtaatc cactaggttt tcatcgtaaa accatcttat gcctgactat 5705
   taaacctatt cttcataaac acaagaacac tttaattttt cgttaattta caaagtaaca 5765
   tcagctgcct atgcctatga taaggtagca gtctgcattc ttatggccat tagatgttac 5825
   aaactccttg cctctaaagt cagatcatga agggataggt gttcatctaa ggttacagtt 5885
   atgttaccga aacacaaaac tgccaaaatc ttactctgct gttatgaatg tttaccatca 5945
   gcattatttt atcatttaat atgtgctcac tgattgttaa ctgtagcttc agcgcgtgcc 6005
   aagcagttga cttaatagga tcatcttgtg aatttgttta cgtgatgcca agcatcaagt 6065
   catgttttct ttagtgtgtg tgcttacaca ggtgttaaac agtttttctc tattttaaac 6125
   tgagccttct ttttaatata ttcccgaaga gatatgtaaa taagctctca gagtttctgt 6185
   gatgatttgt tgagccttgc tggacaagtg gtttgtttgt gtgcaaacca aactttcttt   6245
   acccagtgca atagatttgt ttgactgctt gtgtcttttt atgacctgtt tgccttttag   6305
   aaaattggta aataaagcaa gtatattttt attttta                           6342
<210> 4
   <211> 1255
   <212> PRT
   <213> Homo sapiens
<400> 4
   Met Asn Gly Tyr Ala Glu Phe Pro Pro Ser Pro Ser Asn Pro Thr Lys
   1               5                   10                  15
   Glu Pro Val Glu Pro Gln Pro Ser Gln Val Pro Leu Gln Glu Asp Val
               20                  25                  30
   Asp Met Ser Ser Gly Ser Ser Gly His Glu Thr Asn Glu Asn Cys Ser
           35                  40                  45
   Thr Gly Arg Asp Ser Gln Gly Ser Asp Cys Asp Asp Ser Gly Lys Glu
       50                  55                  60
   Leu Gly Met Leu Val Glu Pro Pro Asp Ala Arg Gln Ser Pro Asp Thr
   65                  70                  75                  80
   Phe Ser Leu Met Met Ala Lys Ser Glu His Asn Pro Ser Thr Ser Gly
                   85                  90                  95
   Cys Ser Ser Asp Gln Ser Ser Lys Val Asp Thr His Lys Glu Leu Ile
               100                 105                 110
   Lys Thr Leu Lys Glu Leu Lys Val His Leu Pro Ala Asp Lys Lys Ala
           115                 120                 125
   Lys Gly Lys Ala Ser Thr Leu Ala Thr Leu Lys Tyr Ala Leu Arg Ser
       130                 135                 140
   Val Lys Gln Val Lys Ala Asn Glu Glu Tyr Tyr Gln Leu Leu Met Ser
   145                 150                 155                 160
   Ser Glu Gly His Pro Cys Gly Ala Asp Val Pro Ser Tyr Thr Val Glu
                   165                 170                 175
   Glu Met Glu Ser Val Thr Ser Glu His Ile Val Lys Asn Ala Asp Met
   180 185 190
   Phe Ala Val Ala Val Ser Leu Val Ser Gly Lys Ile Leu Tyr Ile Ser
   195 200 205
   Asp Gln Val Ala Ser Ile Phe His Cys Lys Arg Asp Ala Phe Ser Asp
   210 215 220
   Ala Lys Phe Val Glu Phe Leu Ala Pro His Asp Val Gly Val Phe His
   225 230 235 240
   Ser Phe Thr Ser Pro Tyr Lys Leu Pro Leu Trp Ser Met Cys Ser Gly
   245 250 255
   Ala Asp Ser Phe Thr Gln Glu Cys Met Glu Glu Lys Ser Phe Phe Cys
   260 265 270
   Arg Val Ser Val Arg Lys Ser His Glu Asn Glu Ile Arg Tyr His Pro
   275 280 285
   Phe Arg Met Thr Pro Tyr Leu Val Lys Val Arg Asp Gln Gln Gly Ala
   290 295 300
   Glu Ser Gln Leu Cys Cys Leu Leu Leu Ala Glu Arg Val His Ser Gly
   305 310 315 320
   Tyr Glu Ala Pro Arg Ile Pro Pro Glu Lys Arg Ile Phe Thr Thr Thr
   325 330 335
   His Thr Pro Asn Cys Leu Phe Gln Asp Val Asp Glu Arg Ala Val Pro
   340 345 350
   Leu Leu Gly Tyr Leu Pro Gln Asp Leu Ile Glu Thr Pro Val Leu Val
   355 360 365
   Gln Leu His Pro Ser Asp Arg Pro Leu Met Leu Ala Ile His Lys Lys
   370 375 380
   Ile Leu Gln Ser Gly Gly Gln Pro Phe Asp Tyr Ser Pro Ile Arg Phe
   385 390 395 400
   Arg Ala Arg Asn Gly Glu Tyr Ile Thr Leu Asp Thr Ser Trp Ser Ser
   405 410 415
   Phe Ile Asn Pro Trp Ser Arg Lys Ile Ser Phe Ile Ile Gly Arg His
   420 425 430
   Lys Val Arg Val Gly Pro Leu Asn Glu Asp Val Phe Ala Ala His Pro
   435 440 445
   Cys Thr Glu Glu Lys Ala Leu His Pro Ser Ile Gln Glu Leu Thr Glu
   450 455 460
   Gln Ile His Arg Leu Leu Leu Gln Pro Val Pro His Ser Gly Ser Ser
   465 470 475 480
   Gly Tyr Gly Ser Leu Gly Ser Asn Gly Ser His Glu His Leu Met Ser
   485 490 495
   Gln Thr Ser Ser Ser Asp Ser Asn Gly His Glu Asp Ser Arg Arg Arg
   500 505 510
   Arg Ala Glu Ile Cys Lys Asn Gly Asn Lys Thr Lys Asn Arg Ser His
   515 520 525
   Tyr Ser His Glu Ser Gly Glu Gln Lys Lys Lys Ser Val Thr Glu Met
   530 535 540
   Gln Thr Asn Pro Pro Ala Glu Lys Lys Ala Val Pro Ala Met Glu Lys
   545 550 555 560
   Asp Ser Leu Gly Val Ser Phe Pro Glu Glu Leu Ala Cys Lys Asn Gln
   565 570 575
   Pro Thr Cys Ser Tyr Gln Gln Ile Ser Cys Leu Asp Ser Val Ile Arg
   580 585 590
   Tyr Leu Glu Ser Cys Asn Glu Ala Ala Thr Leu Lys Arg Lys Cys Glu
   595 600 605
   Phe Pro Ala Asn Val Pro Ala Leu Arg Ser Ser Asp Lys Arg Lys Ala
   610 615 620
   Thr Val Ser Pro Gly Pro His Ala Gly Glu Ala Glu Pro Pro Ser Arg
   625 630 635 640
   Val Asn Ser Arg Thr Gly Val Gly Thr His Leu Thr Ser Leu Ala Leu
   645 650 655
   Pro Gly Lys Ala Glu Ser Val Ala Ser Leu Thr Ser Gln Cys Ser Tyr
   660 665 670
   Ser Ser Thr Ile Val His Val Gly Asp Lys Lys Pro Gln Pro Glu Leu
   675 680 685
   Glu Met Val Glu Asp Ala Ala Ser Gly Pro Glu Ser Leu Asp Cys Leu
   690 695 700
   Ala Gly Pro Ala Leu Ala Cys Gly Leu Ser Gln Glu Lys Glu Pro Phe
   705 710 715 720
   Lys Lys Leu Gly Leu Thr Lys Glu Val Leu Ala Ala His Thr Gln Lys
   725 730 735
   Glu Glu Gln Ser Phe Leu Gln Lys Phe Lys Glu Ile Arg Lys Leu Ser
   740 745 750
   Ile Phe Gln Ser His Cys His Tyr Tyr Leu Gln Glu Arg Ser Lys Gly
   755 760 765
   Gln Pro Ser Glu Arg Thr Ala Pro Gly Leu Arg Asn Thr Ser Gly Ile
   770 775 780
   Asp Ser Pro Trp Lys Lys Thr Gly Lys Asn Arg Lys Leu Lys Ser Lys
   785 790 795 800
   Arg Val Lys Pro Arg Asp Ser Ser Glu Ser Thr Gly Ser Gly Gly Pro
   805 810 815
   Val Ser Ala Arg Pro Pro Leu Val Gly Leu Asn Ala Thr Ala Trp Ser
   820 825 830
   Pro Ser Asp Thr Ser Gln Ser Ser Cys Pro Ala Val Pro Phe Pro Ala
   835 840 845
   Pro Val Pro Ala Ala Tyr Ser Leu Pro Val Phe Pro Ala Pro Gly Thr
   850 855 860
   Val Ala Ala Pro Pro Ala Pro Pro His Ala Ser Phe Thr Val Pro Ala
   865 870 875 880
   Val Pro Val Asp Leu Gln His Gln Phe Ala Val Gln Pro Pro Pro Phe
   885 890 895
   Pro Ala Pro Leu Ala Pro Val Met Ala Phe Met Leu Pro Ser Tyr Ser
   900 905 910
   Phe Pro Ser Gly Thr Pro Asn Leu Pro Gln Ala Phe Phe Pro Ser Gln
   915 920 925
   Pro Gln Phe Pro Ser His Pro Thr Leu Thr Ser Glu Met Ala Ser Ala
   930 935 940
   Ser Gln Pro Glu Phe Pro Ser Arg Thr Ser Ile Pro Arg Gln Pro Cys
   945 950 955 960
   Ala Cys Pro Ala Thr Arg Ala Thr Pro Pro Ser Ala Met Gly Arg Ala
   965 970 975
   Ser Pro Pro Leu Phe Gln Ser Arg Ser Ser Ser Pro Leu Gln Leu Asn
   980 985 990
   Leu Leu Gln Leu Glu Glu Ala Pro Glu Gly Gly Thr Gly Ala Met Gly
   995 1000 1005
   Thr Thr Gly Ala Thr Glu Thr Ala Ala Val Gly Ala Asp Cys Lys
   1010 1015 1020
   Pro Gly Thr Ser Arg Asp Gln Gln Pro Lys Ala Pro Leu Thr Arg
   1025 1030 1035
   Asp Glu Pro Ser Asp Thr Gln Asn Ser Asp Ala Leu Ser Thr Ser
   1040 1045 1050
   Ser Gly Leu Leu Asn Leu Leu Leu Asn Glu Asp Leu Cys Ser Ala
   1055 1060 1065
   Ser Gly Ser Ala Ala Ser Glu Ser Leu Gly Ser Gly Ser Leu Gly
   1070 1075 1080
   Cys Asp Ala Ser Pro Ser Gly Ala Gly Ser Ser Asp Thr Ser His
   1085 1090 1095
   Thr Ser Lys Tyr Phe Gly Ser Ile Asp Ser Ser Glu Asn Asn His
   1100 1105 1110
   Lys Ala Lys Met Asn Thr Gly Met Glu Glu Ser Glu His Phe Ile
   1115 1120 1125
   Lys Cys Val Leu Gln Asp Pro Ile Trp Leu Leu Met Ala Asp Ala
                   1130                    1135                    1140
   Asp Ser Ser Val Met Met Thr Tyr Gln Leu Pro Ser Arg Asn Leu
       1145                    1150                    1155
   Glu Ala Val Leu Lys Glu Asp Arg Glu Lys Leu Lys Leu Leu Gln
       1160                    1165                    1170
   Lys Leu Gln Pro Arg Phe Thr Glu Ser Gln Lys Gln Glu Leu Arg
       1175                    1180                    1185
   Glu Val His Gln Trp Met Gln Thr Gly Gly Leu Pro Ala Ala Ile
       1190                    1195                    1200
   Asp Val Ala Glu Cys Val Tyr Cys Glu Asn Lys Glu Lys Gly Asn
       1205                    1210                    1215
   Ile Cys Ile Pro Tyr Glu Glu Asp Ile Pro Ser Leu Gly Leu Ser
       1220                    1225                    1230
   Glu Val Ser Asp Thr Lys Glu Asp Glu Asn Gly Ser Pro Leu Asn
       1235                    1240                    1245
   His Arg Ile Glu Glu Gln Thr
       1250                    1255
<210> 5
   <211> 2812
   <212> DNA
   <213> Homo sapiens
<220>
   <221> CDS
   <222> (409) .. (2286)
   <223>
<400> 5
   ggaaagtagg ttagtggtgc gacatttagg gaaggcagaa agtaggtcag ggacggaggt     60
   gcctgtttac ccgcgccgga ctcaccgccg ccgccgccgc gggatccgag tgcgggcgcg    120
   ggcgcgggcg ctcccggcga gccacggtgg tgctggctag agtgtatacg tttggaccca    180
   agcttaactt ttccaatgtg gaatcctggg ccttcattgg ttccgatgtc ataggaatct    240
   acgctagagg gcttcctgtg tgccgggcac tgtggttttt ggaagaagct gaccgcctga    300
   aaagaaatta taaaacatga aaatcgcttt gaggtgacca agtccagagg cccctaactc    360
   ctcccaagct ggatctgggg tgtaagaact gtgacttcag atcatcca atg gca gac    417
                                                         Met Ala Asp
                                                             1
   cag aga atg gac att tct tca acc atc agt gat ttc atg tcc ccg ggc       465
   Gln Arg Met Asp Ile Ser Ser Thr Ile Ser Asp Phe Met Ser Pro Gly
       5                   10                  15
   ccc acc gac ctg ctt tcc agc tct ctt ggt acc agt ggt gtg gat tgc       513
   Pro Thr Asp Leu Leu Ser Ser Ser Leu Gly Thr Ser Gly Val Asp Cys
   20                  25                  30                  35
   aac cgc aaa cgg aaa ggc agc tcc act gac tac caa gaa agc atg gac       561
   Asn Arg Lys Arg Lys Gly Ser Ser Thr Asp Tyr Gln Glu Ser Met Asp
                   40                  45                  50
   aca gac aaa gat gac cct cat gga agg tta gaa tat aca gaa cac caa       609
   Thr Asp Lys Asp Asp Pro His Gly Arg Leu Glu Tyr Thr Glu His Gln
               55                  60                  65
   gga agg ata aaa aat gca agg gaa gct cac agt cag att gaa aag cgg       657
   Gly Arg Ile Lys Asn Ala Arg Glu Ala His Ser Gln Ile Glu Lys Arg
           70                  75                  80
   cgt cgg gat aaa atg aac agt ttt ata gat gaa ttg gct tct ttg gta       705
   Arg Arg Asp Lys Met Asn Ser Phe Ile Asp Glu Leu Ala Ser Leu Val
       85                  90                  95
   cca aca tgc aac gca atg tcc agg aaa tta gat aaa ctt act gtg cta       753
   Pro Thr Cys Asn Ala Met Ser Arg Lys Leu Asp Lys Leu Thr Val Leu
   100                 105                 110                 115
   agg atg gct gtt cag cac atg aaa aca tta aga ggt gcc acc aat cca       801
   Arg Met Ala Val Gln His Met Lys Thr Leu Arg Gly Ala Thr Asn Pro
                   120                 125                 130
   tac aca gaa gca aac tac aaa cca act ttt cta tca gac gat gaa ttg       849
   Tyr Thr Glu Ala Asn Tyr Lys Pro Thr Phe Leu Ser Asp Asp Glu Leu
               135                 140                 145
   aaa cac ctc att ctc agg gca gca gat gga ttt ttg ttt gtc gta gga       897
   Lys His Leu Ile Leu Arg Ala Ala Asp Gly Phe Leu Phe Val Val Gly
           150                 155                 160
   tgt gac cga ggg aag ata ctc ttt gtc tca gag tct gtc ttc aag atc       945
   Cys Asp Arg Gly Lys Ile Leu Phe Val Ser Glu Ser Val Phe Lys Ile
       165                 170                 175
   ctc aac tac agc cag aat gat ctg att ggt cag agt ttg ttt gac tac       993
   Leu Asn Tyr Ser Gln Asn Asp Leu Ile Gly Gln Ser Leu Phe Asp Tyr
   180                 185                 190                 195
   ctg cat cct aaa gat att gcc aaa gtc aag gag cag ctc tcc tcc tct      1041
   Leu His Pro Lys Asp Ile Ala Lys Val Lys Glu Gln Leu Ser Ser Ser
                   200                 205                 210
   gac acc gca ccc cgg gag cgg ctc ata gat gca aaa act gga ctt cca      1089
   Asp Thr Ala Pro Arg Glu Arg Leu Ile Asp Ala Lys Thr Gly Leu Pro
               215                 220                 225
   gtt aaa aca gat ata acc cct ggg cca tct cga tta tgt tct gga gca      1137
   Val Lys Thr Asp Ile Thr Pro Gly Pro Ser Arg Leu Cys Ser Gly Ala
           230                 235                 240
   cga cgt tct ttc ttc tgt agg atg aag tgt aac agg cct tca gta aag      1185
   Arg Arg Ser Phe Phe Cys Arg Met Lys Cys Asn Arg Pro Ser Val Lys
       245                 250                 255
   gtt gaa gac aag gac ttc ccc tct acc tgc tca aag aaa aaa gat cga      1233
   Val Glu Asp Lys Asp Phe Pro Ser Thr Cys Ser Lys Lys Lys Asp Arg
   260                 265                 270                 275
   aaa agc ttc tgc aca atc cac agc aca ggc tat ttg aaa agc tgg cca      1281
   Lys Ser Phe Cys Thr Ile His Ser Thr Gly Tyr Leu Lys Ser Trp Pro
                   280                 285                 290
   ccc aca aag atg ggg ctg gat gaa gac aac gaa cca gac aat gag ggg      1329
   Pro Thr Lys Met Gly Leu Asp Glu Asp Asn Glu Pro Asp Asn Glu Gly
               295                 300                 305
   tgt aac ctc agc tgc ctc gtc gca att gga cga ctg cat tct cat gta      1377
   Cys Asn Leu Ser Cys Leu Val Ala Ile Gly Arg Leu His Ser His Val
           310                 315                 320
   gtt cca caa cca gtg aac ggg gaa atc agg gtg aaa tct atg gaa tat      1425
   Val Pro Gln Pro Val Asn Gly Glu Ile Arg Val Lys Ser Met Glu Tyr
       325                 330                 335
   gtt tct cgg cac gcg ata gat gga aag ttt gtt ttt gta gac cag agg      1473
   Val Ser Arg His Ala Ile Asp Gly Lys Phe Val Phe Val Asp Gln Arg
   340                 345                 350                 355
   gca aca gct att ttg gca tat tta cca caa gaa ctt cta ggc aca tcg      1521
   Ala Thr Ala Ile Leu Ala Tyr Leu Pro Gln Glu Leu Leu Gly Thr Ser
                   360                 365                 370
   tgt tat gaa tat ttt cac caa gat gac ata gga cat ctt gca gaa tgt      1569
   Cys Tyr Glu Tyr Phe His Gln Asp Asp Ile Gly His Leu Ala Glu Cys
               375                 380                 385
   cat agg caa gtt tta cag acg aga gaa aaa att aca act aat tgc tat      1617
   His Arg Gln Val Leu Gln Thr Arg Glu Lys Ile Thr Thr Asn Cys Tyr
           390                 395                 400
   aaa ttt aaa atc aaa gat ggt tct ttt atc aca cta cgg agt cga tgg      1665
   Lys Phe Lys Ile Lys Asp Gly Ser Phe Ile Thr Leu Arg Ser Arg Trp
       405                 410                 415
   ttc agt ttc atg aac cct tgg acc aag gaa gta gaa tat att gtc tca      1713
   Phe Ser Phe Met Asn Pro Trp Thr Lys Glu Val Glu Tyr Ile Val Ser
   420                 425                 430                 435
   act aac act gtt gtt tta gcc aac gtc ctg gaa ggc ggg gac cca acc      1761
   Thr Asn Thr Val Val Leu Ala Asn Val Leu Glu Gly Gly Asp Pro Thr
                   440                 445                 450
   ttc cca cag ctc aca gca tcc ccc cac agc atg gac agc atg ctg ccc      1809
   Phe Pro Gln Leu Thr Ala Ser Pro His Ser Met Asp Ser Met Leu Pro
               455                 460                 465
   tct gga gaa ggt ggc cca aag agg acc cac ccc act gtt cca ggg att      1857
   Ser Gly Glu Gly Gly Pro Lys Arg Thr His Pro Thr Val Pro Gly Ile
           470                 475                 480
   cca ggg gga acc cgg gct ggg gca gga aaa ata ggc cga atg att gct      1905
   Pro Gly Gly Thr Arg Ala Gly Ala Gly Lys Ile Gly Arg Met Ile Ala
       485                 490                 495
   gag gaa atc atg gaa atc cac agg ata aga ggg tca tcg cct tct agc      1953
   Glu Glu Ile Met Glu Ile His Arg Ile Arg Gly Ser Ser Pro Ser Ser
   500                 505                 510                 515
   tgt ggc tcc agc cca ttg aac atc acg agt acg cct ccc cct gat gcc      2001
   Cys Gly Ser Ser Pro Leu Asn Ile Thr Ser Thr Pro Pro Pro Asp Ala
                   520                 525                 530
   tct tct cca gga ggc aag aag att tta aat gga ggg act cca gac att      2049
   Ser Ser Pro Gly Gly Lys Lys Ile Leu Asn Gly Gly Thr Pro Asp Ile
               535                 540                 545
   cct tcc agt ggc cta cta tca ggc cag gct cag gag aac cca ggt tat      2097
   Pro Ser Ser Gly Leu Leu Ser Gly Gln Ala Gln Glu Asn Pro Gly Tyr
           550                 555                 560
   cca tat tct gat agt tct tct att ctt ggt gag aac ccc cac ata ggt      2145
   Pro Tyr Ser Asp Ser Ser Ser Ile Leu Gly Glu Asn Pro His Ile Gly
       565                 570                 575
   ata gac atg att gac aac gac caa gga tca agt agt ccc agt aat gat      2193
   Ile Asp Met Ile Asp Asn Asp Gln Gly Ser Ser Ser Pro Ser Asn Asp
   580                 585                 590                 595
   gag gca gca atg gct gtc atc atg agc ctc ttg gaa gca gat gct gga      2241
   Glu Ala Ala Met Ala Val Ile Met Ser Leu Leu Glu Ala Asp Ala Gly
                   600                 605                 610
   ctg ggt ggc cct gtt gac ttt agt gac ttg cca tgg ccg ctg taa          2286
   Leu Gly Gly Pro Val Asp Phe Ser Asp Leu Pro Trp Pro Leu
               615                 620                 625
   acactacatg ttgctttggc aacagctata gtatcaaagt gcattactgg tggagtttta    2346
   cagtctgtga agcttactgg ataaggagag aatagctttt atgtactgac ttcataaaag    2406
   ccatctcaga gccattgata caagtcaatc ttactatatg taacttcaga caaagtggaa    2466
   ctaagcctgc tccagtgttt cctcatcatt gattattggg ctagctgtgg atagcttgca    2526
   ttaattgtat attttggatt ctgtttgtgt tgaatttttt aatcattgtg cacagaagca    2586
   tcattggtag cttttatatg caaatggtca tttcagatgt atggtgtttt tacactacaa    2646
   agaagtcccc catgtggata tttcttatac taattgtatc ataaagccgt ttattcttcc    2706
   ttgtaagaat cctttactat aaatatgggt taaagtataa tgtactagac agttaaatat    2766
   ttttaataaa tgtttccctt gttctataaa aaaaaaaaaa aaaaaa                   2812
<210> 6
   <211> 625
   <212> PRT
   <213> Homo sapiens
<400> 6
   Met Ala Asp Gln Arg Met Asp Ile Ser Ser Thr Ile Ser Asp Phe Met
   1               5                   10                  15
   Ser Pro Gly Pro Thr Asp Leu Leu Ser Ser Ser Leu Gly Thr Ser Gly
               20                  25                  30
   Val Asp Cys Asn Arg Lys Arg Lys Gly Ser Ser Thr Asp Tyr Gln Glu
           35                  40                  45
   Ser Met Asp Thr Asp Lys Asp Asp Pro His Gly Arg Leu Glu Tyr Thr
       50                  55                  60
   Glu His Gln Gly Arg Ile Lys Asn Ala Arg Glu Ala His Ser Gln Ile
   65                  70                  75                  80
   Glu Lys Arg Arg Arg Asp Lys Met Asn Ser Phe Ile Asp Glu Leu Ala
                   85                  90                  95
   Ser Leu Val Pro Thr Cys Asn Ala Met Ser Arg Lys Leu Asp Lys Leu
               100                 105                 110
   Thr Val Leu Arg Met Ala Val Gln His Met Lys Thr Leu Arg Gly Ala
           115                 120                 125
   Thr Asn Pro Tyr Thr Glu Ala Asn Tyr Lys Pro Thr Phe Leu Ser Asp
       130                 135                 140
   Asp Glu Leu Lys His Leu Ile Leu Arg Ala Ala Asp Gly Phe Leu Phe
   145                 150                 155                 160
   Val Val Gly Cys Asp Arg Gly Lys Ile Leu Phe Val Ser Glu Ser Val
                   165                 170                 175
   Phe Lys Ile Leu Asn Tyr Ser Gln Asn Asp Leu Ile Gly Gln Ser Leu
               180                 185                 190
   Phe Asp Tyr Leu His Pro Lys Asp Ile Ala Lys Val Lys Glu Gln Leu
           195                 200                 205
   Ser Ser Ser Asp Thr Ala Pro Arg Glu Arg Leu Ile Asp Ala Lys Thr
       210                 215                 220
   Gly Leu Pro Val Lys Thr Asp Ile Thr Pro Gly Pro Ser Arg Leu Cys
   225                 230                 235                 240
   Ser Gly Ala Arg Arg Ser Phe Phe Cys Arg Met Lys Cys Asn Arg Pro
                   245                 250                 255
   Ser Val Lys Val Glu Asp Lys Asp Phe Pro Ser Thr Cys Ser Lys Lys
               260                 265                 270
   Lys Asp Arg Lys Ser Phe Cys Thr Ile His Ser Thr Gly Tyr Leu Lys
           275                 280                 285
   Ser Trp Pro Pro Thr Lys Met Gly Leu Asp Glu Asp Asn Glu Pro Asp
       290                 295                 300
   Asn Glu Gly Cys Asn Leu Ser Cys Leu Val Ala Ile Gly Arg Leu His
   305                 310                 315                 320
   Ser His Val Val Pro Gln Pro Val Asn Gly Glu Ile Arg Val Lys Ser
                   325                 330                 335
   Met Glu Tyr Val Ser Arg His Ala Ile Asp Gly Lys Phe Val Phe Val
               340                 345                 350
   Asp Gln Arg Ala Thr Ala Ile Leu Ala Tyr Leu Pro Gln Glu Leu Leu
           355                 360                 365
   Gly Thr Ser Cys Tyr Glu Tyr Phe His Gln Asp Asp Ile Gly His Leu
       370                 375                 380
   Ala Glu Cys His Arg Gln Val Leu Gln Thr Arg Glu Lys Ile Thr Thr
   385                 390                 395                 400
   Asn Cys Tyr Lys Phe Lys Ile Lys Asp Gly Ser Phe Ile Thr Leu Arg
                   405                 410                 415
   Ser Arg Trp Phe Ser Phe Met Asn Pro Trp Thr Lys Glu Val Glu Tyr
               420                 425                 430
   Ile Val Ser Thr Asn Thr Val Val Leu Ala Asn Val Leu Glu Gly Gly
           435                 440                 445
   Asp Pro Thr Phe Pro Gln Leu Thr Ala Ser Pro His Ser Met Asp Ser
       450                 455                 460
   Met Leu Pro Ser Gly Glu Gly Gly Pro Lys Arg Thr His Pro Thr Val
   465 470 475 480
   Pro Gly Ile Pro Gly Gly Thr Arg Ala Gly Ala Gly Lys Ile Gly Arg
   485 490 495
   Met Ile Ala Glu Glu Ile Met Glu Ile His Arg Ile Arg Gly Ser Ser
   500 505 510
   Pro Ser Ser Cys Gly Ser Ser Pro Leu Asn Ile Thr Ser Thr Pro Pro
   515 520 525
   Pro Asp Ala Ser Ser Pro Gly Gly Lys Lys Ile Leu Asn Gly Gly Thr
   530 535 540
   Pro Asp Ile Pro Ser Ser Gly Leu Leu Ser Gly Gln Ala Gln Glu Asn
   545 550 555 560
   Pro Gly Tyr Pro Tyr Ser Asp Ser Ser Ser Ile Leu Gly Glu Asn Pro
   565 570 575
   His Ile Gly Ile Asp Met Ile Asp Asn Asp Gln Gly Ser Ser Ser Pro
   580 585 590
   Ser Asn Asp Glu Ala Ala Met Ala Val Ile Met Ser Leu Leu Glu Ala
   595 600 605
   Asp Ala Gly Leu Gly Gly Pro Val Asp Phe Ser Asp Leu Pro Trp Pro
   610 615 620
   Leu
   625
<210> 7
   <211> 3935
   <212> DNA
   <213> Homo sapiens
<220>
   <221> CDS
   <222> (251)..(2725)
   <223>
<400> 7
   gagggacccg cgcggctgcg gcccaggagc ggcggccgcg gagcccggag acccgcagcc 60
   gcggcggcgg cggcggcggc ggcagcagct agagcagcgc ctcccgccgc cgcccgggag 120
   gagctcgccg cgcccgctcg ccgcctcgtc tcccagcggc ggcgggaggc gcgtctcccc 180
   ggcccagtcc gcgcccggcc ccgcggggcc gctccggccc gctccgagga aaaactgcat      240
   agaaaatcta atg gat gaa gat gag aaa gac aga gcc aag aga gct tct         289
   cga aac aag tct gag aag aag cgt cgg gac cag ttc aat gtt ctc atc        337
   aaa gag ctc agt tcc atg ctc cct ggc aac acg cgg aaa atg gac aaa        385
   acc acc gtg ttg gaa aag gtc atc gga ttt ttg cag aaa cac aat gaa        433
   gtc tca gcg caa acg gaa atc tgt gac att cag caa gac tgg aag cct        481
   tca ttc ctc agt aat gaa gaa ttc acc cag ctg atg ttg gag gca tta        529
   gat ggc ttc att atc gca gtg aca aca gac ggc agc atc atc tat gtc        577
   tct gac agt atc acg cct ctc ctt ggg cat tta ccg tcg gat gtc atg        625
   gat cag aat ttg tta aat ttc ctc cca gaa caa gaa cat tca gaa gtt        673
   tat aaa atc ctt tct tcc cat atg ctt gtg acg gat tcc ccc tcc cca        721
   gaa tac tta aaa tct gac agc gat tta gag ttt tat tgc cat ctt ctc        769
   aga ggc agc ttg aac cca aag gaa ttt cca act tat gaa tac ata aaa        817
   ttt gta gga aat ttt cgc tct tac aac aat gtg cct agc ccc tcc tgt        865
   aat ggt ttt gac aac acc ctt tca aga cct tgc cgg gtg cca cta gga        913
   aag gag gtt tgc ttc att gcc acc gtt cgt ctg gca aca cca caa ttc        961
   tta aag gaa atg tgc ata gtt gac gaa cct tta gag gaa ttc act tca      1009
   agg cat agc ttg gaa tgg aaa ttt tta ttt ctg gat cac aga gca cct      1057
   cca atc ata gga tac ctg cct ttt gaa gtg ctg gga acc tca ggc tat      1105
   gac tac tac cac att gat gac ctg gag ctc ctg gcc agg tgt cac cag      1153
   cac ctg atg cag ttt ggc aaa ggg aag tcg tgt tgc tac cgg ttt ctg      1201
   acc aaa ggt cag cag tgg atc tgg ctg cag act cac tac tac atc acc      1249
   tac cat cag tgg aac tcc aag ccc gag ttc atc gtg tgc aca cac tcg      1297
   gtg gtc agt tac gca gat gtc cgg gtg gaa agg agg cag gag ctg gct      1345
   ctg gaa gac ccg cca tcc gag gcc ctc cac tcc tca gca cta aag gac      1393
   aag ggc tca agc ctg gaa cct cgg cag cac ttt aac gca ctc gac gtg      1441
   ggt gcc tcg ggc ctt aat acc agt cat tcg cca tcg gcg tcc tca aga      1489
   agt tcc cac aaa tcc tcg cac aca gcc atg tca gaa ccc acc tcc act      1537
   ccc acc aag ctg atg gca gag gcc agc acc ccg gct ttg cca aga tca      1585
   gcc acc ctg ccc caa gag tta cct gtc ccc ggg ctc agc cag gca gcc      1633
   acc atg ccg gcc cct ctg cct tcc cca tcg tcc tgc gac ctc aca cag      1681
   cag ctc ctg cct cag acc gtt ctg cag agc acg ccc gct ccc atg gca      1729
   cag ttt tcg gca cag ttc agc atg ttc cag acc atc aaa gac cag cta      1777
   gag cag cgg acg cgg atc ctg cag gcc aat atc cgg tgg caa cag gaa      1825
   gag ctc cac aag atc cag gag cag ctc tgc ctg gtc cag gac tcc aac      1873
   gtc cag atg ttc ctg cag cag cca gct gta tcc ctg agc ttc agc agc      1921
   acc cag cga cct gag gct cag cag cag cta cag caa agg tca gct gca      1969
   gtg act cag ccc cag ctc ggg gcg ggc ccc caa ctt cca ggg cag atc      2017
   tcc tct gcc cag gtc aca agc cag cac ctg ctc aga gaa tca agt gtg      2065
   ata tca acc cag ggt cca aag cca atg aga agc tca cag cta atg cag      2113
   agc agc ggc cgc tct gga agc agc cta gtg tcc ccg ttc agc agc gcc      2161
   aca gct gcg ctc ccg cca agt ctg aat ctg acc aca cct gct tcc acc      2209
   tcc cag gat gcc agc cag tgc cag ccc agc cca gac ttc agc cat gat      2257
   cgg cag ctc agg ctg ttg ctg agc cag ccc atc cag ccc atg atg ccc      2305
   ggg tcc tgt gac gca agg cag ccc tcg gaa gtc agc agg acg gga cgg      2353
   caa gtc aag tac gcc cag agc cag act gtg ttt caa aat cca gac gca      2401
   cac ccc gcc aac agc agc agc gcc ccg atg ccc gtc ctg ctg atg ggg      2449
   cag gcg gtg ctc cac ccc agc ttc cct gcc tcc caa cca tcg ccc ctg      2497
   cag cct gca cag gcc cgg cag cag cca ccg cag cac tac ctg cag gta      2545
   cag gca cca acc tct ttg cac agt gag cag cag gac tcg cta ctt ctc      2593
   tcc acc tac tca caa cag cca ggg acc ctg ggc tac ccc caa cca ccc      2641
   cca gca cag ccc cag ccc cta cgt cct ccc cga agg gtc agc agt ctg      2689
   tct gag tcg tca ggc ctc cag cag ccg ccc cga taa tgccccggca           2735
   ctgaagtcgg gacacaatca gctttaacca atggatgagg ggggtggcca caggagatgg    2795
   ggagaggagt ctgaactaaa cccctggctt ttgtgcacac tgcatacgtt tcagaactcc    2855
   tggatggtaa ccatctctgg agtgcagcgc ttgctgcagt ggaaatgatc aggaatactg    2915
   accgtgtttc tcttgcctcc gaggttcttg ggcacactct atagccatac tggacaggaa    2975
   ccaggtgccc cgtgtaggca tcgtcggtcg gtttgccgtc agagatggcg catctcgctg    3035
   catcccccga gagtacaccg gttgctctag ccacctgcgg cccgcccatc tgcgctagct    3095
   ggccttcacg ctcttgatcg tctttccttt gtattggaga aggactgggt cagagatctg    3155
   ttggagagag agaataaaga gattattttt cattattttt aaatggttgt ttttgtttta    3215
   atttgcacag ctacacagag gaaataactt aggtactttc tgttttttta aaaaaaataa    3275
   taaggtctca tggcttcatt cagagaccac agtaacaaca gcagcccacc aatcagagaa    3335
   gctggttgtt attaaccaag ctacagattc acactttctg gcctaaaccc taatgggatg    3395
   aggcttttca ccccaggcca tgctggtggt gattttttag cccctaaata aaacactgga    3455
   ctatttcctg tttacttcat tgattgcaac tacaaaggtg gactcaaagc aaagcacaat    3515
   catgccagcc aacattccag aattctgctg agaactccaa gtctgtgagg ggagaggttt    3575
   tacaagccag acaggcctgg gggactgcag tccccaagga gaccctgcca catgctggcc    3635
   ctttgagtga gaatgctgca tctttctaca tatcttcatg agaatactga gaattggatt 3695
   ttccttttca aaatgcactt tgcttttttt gtatgttttg ttatgttgag atgtttctaa 3755
   agaaaagatt ttatgtaatt ataagatgaa gcgtagtgaa ttgtacagct gttgtaataa 3815
   tgacctattt ctatataaaa taaaattgta tggcttatgt gtaaattatt ttgtatctga 3875
   gataccagtt ccttttccca aatataaaag tataaaagtt aaaaaaaaaa aaaaaaaaaa 3935
<210> 8
   <211> 824
   <212> PRT
   <213> Homo sapiens
<400> 8
<210> 9
   <211> 2766
   <212> DNA
   <213> Homo sapiens
<220>
   <221> CDS
   <222> (627)..(2471)
   <223>
<400> 9
   cgaggcgctc cctgggatca catggtacct gctccagtgc cgcgtgcggc ccgggaaccc      60
   tgggctgctg gcgcctgcgc agagccctct gtcccaggga aaggctcggg caaaaggcgg    120
   ctgagattgg cagagtgaaa tattactgcc gagggaacgt agcagggcac acgtctcgcc    180
   tctttgcgac tcggtgcccc gtttctcccc atcacctact tacttcctgg ttgcaacctc    240
   tcttcctctg ggacttttgc accgggagct ccagattcgc taccccgcag cgctgcggag    300
   ccggcaggca gaggcacccc gtacactgca gagacccgac cctccttgct accttctagc    360
   cagaactact gcaggctgat tccccctaca cactctctct gctcttccca tgcaaagcag    420
   aactccgttg cctcaacgtc caacccttct gcagggctgc agtccggcca ccccaagacc    480
   ttgctgcagg gtgcttcgga tcctgatcgt gagtcgcggg gtccactccc cgcccttagc    540
   cagtgcccag gggcaacagc cggcgatcgc aacctctagt ttgagtcaag gtccagtttg     600
   aatgaccgct ctcagctggt gaagac atg acg acc ctg gac tcc aac aac aac     653
                                Met Thr Thr Leu Asp Ser Asn Asn Asn
                                1               5
   aca ggt ggc gtc atc acc tac att ggc tcc agt ggc tcc tcc cca agc     701
   Thr Gly Gly Val Ile Thr Tyr Ile Gly Ser Ser Gly Ser Ser Pro Ser
   10                  15                  20                  25
   cgc acc agc cct gaa tcc ctc tat agt gac aac tcc aat ggc agc ttc     749
   Arg Thr Ser Pro Glu Ser Leu Tyr Ser Asp Asn Ser Asn Gly Ser Phe
                   30                  35                  40
   cag tcc ctg acc caa ggc tgt ccc acc tac ttc cca cca tcc ccc act     797
   Gln Ser Leu Thr Gln Gly Cys Pro Thr Tyr Phe Pro Pro Ser Pro Thr
               45                  50                  55
   ggc tcc ctc acc caa gac ccg gct cgc tcc ttt ggg agc att cca ccc     845
   Gly Ser Leu Thr Gln Asp Pro Ala Arg Ser Phe Gly Ser Ile Pro Pro
           60                  65                  70
   agc ctg agt gat gac ggc tcc cct tct tcc tca tct tcc tcg tcg tca     893
   Ser Leu Ser Asp Asp Gly Ser Pro Ser Ser Ser Ser Ser Ser Ser Ser
       75                  80                  85
   tcc tcc tcc tcc ttc tat aat ggg agc ccc cct ggg agt cta caa gtg     941
   Ser Ser Ser Ser Phe Tyr Asn Gly Ser Pro Pro Gly Ser Leu Gln Val
   90                  95                  100                 105
   gcc atg gag gac agc agc cga gtg tcc ccc agc aag agc acc agc aac     989
   Ala Met Glu Asp Ser Ser Arg Val Ser Pro Ser Lys Ser Thr Ser Asn
                   110                 115                 120
   atc acc aag ctg aat ggc atg gtg tta ctg tgt aaa gtg tgt ggg gac    1037
   Ile Thr Lys Leu Asn Gly Met Val Leu Leu Cys Lys Val Cys Gly Asp
               125                 130                 135
   gtt gcc tcg ggc ttc cac tac ggt gtg cac gcc tgc gag ggc tgc aag    1085
   Val Ala Ser Gly Phe His Tyr Gly Val His Ala Cys Glu Gly Cys Lys
           140                 145                 150
   ggc ttt ttc cgt cgg agc atc cag cag aac atc cag tac aaa agg tgt    1133
   Gly Phe Phe Arg Arg Ser Ile Gln Gln Asn Ile Gln Tyr Lys Arg Cys
           155                 160                 165
   ctg aag aat gag aat tgc tcc atc gtc cgc atc aat cgc aac cgc tgc       1181
   Leu Lys Asn Glu Asn Cys Ser Ile Val Arg Ile Asn Arg Asn Arg Cys
   170                 175                 180                 185
   cag caa tgt cgc ttc aag aag tgt ctc tct gtg ggc atg tct cga gac       1229
   Gln Gln Cys Arg Phe Lys Lys Cys Leu Ser Val Gly Met Ser Arg Asp
                   190                 195                 200
   gct gtg cgt ttt ggg cgc atc ccc aaa cga gag aag cag cgg atg ctt       1277
   Ala Val Arg Phe Gly Arg Ile Pro Lys Arg Glu Lys Gln Arg Met Leu
               205                 210                 215
   gct gag atg cag agt gcc atg aac ctg gcc aac aac cag ttg agc agc       1325
   Ala Glu Met Gln Ser Ala Met Asn Leu Ala Asn Asn Gln Leu Ser Ser
           220                 225                 230
   cag tgc ccg ctg gag act tca ccc acc cag cac ccc acc cca ggc ccc       1373
   Gln Cys Pro Leu Glu Thr Ser Pro Thr Gln His Pro Thr Pro Gly Pro
       235                 240                 245
   atg ggc ccc tcg cca ccc cct gct ccg gtc ccc tca ccc ctg gtg ggc       1421
   Met Gly Pro Ser Pro Pro Pro Ala Pro Val Pro Ser Pro Leu Val Gly
   250                 255                 260                 265
   ttc tcc cag ttt cca caa cag ctg acg cct ccc aga tcc cca agc cct       1469
   Phe Ser Gln Phe Pro Gln Gln Leu Thr Pro Pro Arg Ser Pro Ser Pro
                   270                 275                 280
   gag ccc aca gtg gag gat gtg ata tcc cag gtg gcc cgg gcc cat cga       1517
   Glu Pro Thr Val Glu Asp Val Ile Ser Gln Val Ala Arg Ala His Arg
               285                 290                 295
   gag atc ttc acc tac gcc cat gac aag ctg ggc agc tca cct ggc aac       1565
   Glu Ile Phe Thr Tyr Ala His Asp Lys Leu Gly Ser Ser Pro Gly Asn
           300                 305                 310
   ttc aat gcc aac cat gca tca ggt agc cct cca gcc acc acc cca cat       1613
   Phe Asn Ala Asn His Ala Ser Gly Ser Pro Pro Ala Thr Thr Pro His
       315                 320                 325
   cgc tgg gaa aat cag ggc tgc cca cct gcc ccc aat gac aac aac acc       1661
   Arg Trp Glu Asn Gln Gly Cys Pro Pro Ala Pro Asn Asp Asn Asn Thr
   330                 335                 340                 345
   ttg gct gcc cag cgt cat aac gag gcc cta aat ggt ctg cgc cag gct       1709
   Leu Ala Ala Gln Arg His Asn Glu Ala Leu Asn Gly Leu Arg Gln Ala
                   350                 355                 360
   ccc tcc tcc tac cct ccc acc tgg cct cct ggc cct gca cac cac agc       1757
   Pro Ser Ser Tyr Pro Pro Thr Trp Pro Pro Gly Pro Ala His His Ser
               365                 370                 375
   tgc cac cag tcc aac agc aac ggg cac cgt cta tgc ccc acc cac gtg       1805
   Cys His Gln Ser Asn Ser Asn Gly His Arg Leu Cys Pro Thr His Val
           380                 385                 390
   tat gca gcc cca gaa ggc aag gca cct gcc aac agt ccc cgg cag ggc       1853
   Tyr Ala Ala Pro Glu Gly Lys Ala Pro Ala Asn Ser Pro Arg Gln Gly
   395                 400                 405
   aac tca aag aat gtt ctg ctg gca tgt cct atg aac atg tac ccg cat      1901
   Asn Ser Lys Asn Val Leu Leu Ala Cys Pro Met Asn Met Tyr Pro His
   410                 415                 420                 425
   gga cgc agt ggg cga acg gtg cag gag atc tgg gag gat ttc tcc atg      1949
   Gly Arg Ser Gly Arg Thr Val Gln Glu Ile Trp Glu Asp Phe Ser Met
                   430                 435                 440
   agc ttc acg ccc gct gtg cgg gag gtg gta gag ttt gcc aaa cac atc      1997
   Ser Phe Thr Pro Ala Val Arg Glu Val Val Glu Phe Ala Lys His Ile
               445                 450                 455
   ccg ggc ttc cgt gac ctt tct cag cat gac caa gtc acc ctg ctt aag      2045
   Pro Gly Phe Arg Asp Leu Ser Gln His Asp Gln Val Thr Leu Leu Lys
           460                 465                 470
   gct ggc acc ttt gag gtg ctg atg gtg cgc ttt gct tcg ttg ttc aac      2093
   Ala Gly Thr Phe Glu Val Leu Met Val Arg Phe Ala Ser Leu Phe Asn
       475                 480                 485
   gtg aag gac cag aca gtg atg ttc cta agc cgc acc acc tac agc ctg      2141
   Val Lys Asp Gln Thr Val Met Phe Leu Ser Arg Thr Thr Tyr Ser Leu
   490                 495                 500                 505
   cag gag ctt ggt gcc atg ggc atg gga gac ctg ctc agt gcc atg ttc      2189
   Gln Glu Leu Gly Ala Met Gly Met Gly Asp Leu Leu Ser Ala Met Phe
                   510                 515                 520
   gac ttc agc gag aag ctc aac tcc ctg gcg ctt acc gag gag gag ctg      2237
   Asp Phe Ser Glu Lys Leu Asn Ser Leu Ala Leu Thr Glu Glu Glu Leu
               525                 530                 535
   ggc ctc ttc acc gcg gtg gtg ctt gtc tct gca gac cgc tcg ggc atg      2285
   Gly Leu Phe Thr Ala Val Val Leu Val Ser Ala Asp Arg Ser Gly Met
           540                 545                 550
   gag aat tcc gct tcg gtg gag cag ctc cag gag acg ctg ctg cgg gct      2333
   Glu Asn Ser Ala Ser Val Glu Gln Leu Gln Glu Thr Leu Leu Arg Ala
       555                 560                 565
   ctt cgg gct ctg gtg ctg aag aac cgg ccc ttg gag act tcc cgc ttc      2381
   Leu Arg Ala Leu Val Leu Lys Asn Arg Pro Leu Glu Thr Ser Arg Phe
   570                 575                 580                 585
   acc aag ctg ctg ctc aag ctg ccg gac ctg cgg acc ctg aac aac atg      2429
   Thr Lys Leu Leu Leu Lys Leu Pro Asp Leu Arg Thr Leu Asn Asn Met
                   590                 595                 600
   cat tcc gag aag ctg ctg tcc ttc cgg gtg gac gcc cag tga              2471
   His Ser Glu Lys Leu Leu Ser Phe Arg Val Asp Ala Gln
               605                 610
   cccgcccggc cggccttctg ccgctgcccc cttgtacaga atcgaactct gcacttctct    2531
   ctcctttacg agacgaaaag gaaaagcaaa ccagaatctt atttatattg ttataaaata    2591
   ttccaagatg agcctctggc cccctgagcc ttcttgtaaa tacctgcctc cctcccccat    2651
   caccgaactt cccctcctcc cctatttaaa ccactctgtc tcccccacaa ccctcccctg    2711
   gccctctgat ttgttctgtt cctgtctcaa atccaatagt tcacagctga gctgg         2766
<210> 10
   <211> 614
   <212> PRT
   <213> Homo sapiens
<400> 10
   Met Thr Thr Leu Asp Ser Asn Asn Asn Thr Gly Gly Val Ile Thr Tyr
   1               5                   10                  15
   Ile Gly Ser Ser Gly Ser Ser Pro Ser Arg Thr Ser Pro Glu Ser Leu
               20                  25                  30
   Tyr Ser Asp Asn Ser Asn Gly Ser Phe Gln Ser Leu Thr Gln Gly Cys
           35                  40                  45
   Pro Thr Tyr Phe Pro Pro Ser Pro Thr Gly Ser Leu Thr Gln Asp Pro
       50                  55                  60
   Ala Arg Ser Phe Gly Ser Ile Pro Pro Ser Leu Ser Asp Asp Gly Ser
   65                  70                  75                  80
   Pro Ser Ser Ser Ser Ser Ser Ser Ser Ser Ser Ser Ser Phe Tyr Asn
                   85                  90                  95
   Gly Ser Pro Pro Gly Ser Leu Gln Val Ala Met Glu Asp Ser Ser Arg
               100                 105                 110
   Val Ser Pro Ser Lys Ser Thr Ser Asn Ile Thr Lys Leu Asn Gly Met
           115                 120                 125
   Val Leu Leu Cys Lys Val Cys Gly Asp Val Ala Ser Gly Phe His Tyr
       130                 135                 140
   Gly Val His Ala Cys Glu Gly Cys Lys Gly Phe Phe Arg Arg Ser Ile
   145                 150                 155                 160
   Gln Gln Asn Ile Gln Tyr Lys Arg Cys Leu Lys Asn Glu Asn Cys Ser
                   165                 170                 175
   Ile Val Arg Ile Asn Arg Asn Arg Cys Gln Gln Cys Arg Phe Lys Lys
               180                 185                 190
   Cys Leu Ser Val Gly Met Ser Arg Asp Ala Val Arg Phe Gly Arg Ile
           195                 200                 205
   Pro Lys Arg Glu Lys Gln Arg Met Leu Ala Glu Met Gln Ser Ala Met
       210                 215                 220
   Asn Leu Ala Asn Asn Gln Leu Ser Ser Gln Cys Pro Leu Glu Thr Ser
   225                 230                 235                 240
   Pro Thr Gln His Pro Thr Pro Gly Pro Met Gly Pro Ser Pro Pro Pro
                   245                 250                 255
   Ala Pro Val Pro Ser Pro Leu Val Gly Phe Ser Gln Phe Pro Gln Gln
               260                 265                 270
   Leu Thr Pro Pro Arg Ser Pro Ser Pro Glu Pro Thr Val Glu Asp Val
           275                 280                 285
   Ile Ser Gln Val Ala Arg Ala His Arg Glu Ile Phe Thr Tyr Ala His
       290                 295                 300
   Asp Lys Leu Gly Ser Ser Pro Gly Asn Phe Asn Ala Asn His Ala Ser
   305                 310                 315                 320
   Gly Ser Pro Pro Ala Thr Thr Pro His Arg Trp Glu Asn Gln Gly Cys
                   325                 330                 335
   Pro Pro Ala Pro Asn Asp Asn Asn Thr Leu Ala Ala Gln Arg His Asn
               340                 345                 350
   Glu Ala Leu Asn Gly Leu Arg Gln Ala Pro Ser Ser Tyr Pro Pro Thr
           355                 360                 365
   Trp Pro Pro Gly Pro Ala His His Ser Cys His Gln Ser Asn Ser Asn
       370                 375                 380
   Gly His Arg Leu Cys Pro Thr His Val Tyr Ala Ala Pro Glu Gly Lys
   385                 390                 395                 400
   Ala Pro Ala Asn Ser Pro Arg Gln Gly Asn Ser Lys Asn Val Leu Leu
                   405                 410                 415
   Ala Cys Pro Met Asn Met Tyr Pro His Gly Arg Ser Gly Arg Thr Val
               420                 425                 430
   Gln Glu Ile Trp Glu Asp Phe Ser Met Ser Phe Thr Pro Ala Val Arg
           435                 440                 445
   Glu Val Val Glu Phe Ala Lys His Ile Pro Gly Phe Arg Asp Leu Ser
       450                 455                 460
   Gln His Asp Gln Val Thr Leu Leu Lys Ala Gly Thr Phe Glu Val Leu
   465                 470                 475                 480
   Met Val Arg Phe Ala Ser Leu Phe Asn Val Lys Asp Gln Thr Val Met
                   485                 490                 495
   Phe Leu Ser Arg Thr Thr Tyr Ser Leu Gln Glu Leu Gly Ala Met Gly
               500                 505                 510
   Met Gly Asp Leu Leu Ser Ala Met Phe Asp Phe Ser Glu Lys Leu Asn
           515                 520                 525
   Ser Leu Ala Leu Thr Glu Glu Glu Leu Gly Leu Phe Thr Ala Val Val
       530                 535                 540
   Leu Val Ser Ala Asp Arg Ser Gly Met Glu Asn Ser Ala Ser Val Glu
   545                 550                 555                 560
   Gln Leu Gln Glu Thr Leu Leu Arg Ala Leu Arg Ala Leu Val Leu Lys
                   565                 570                 575
   Asn Arg Pro Leu Glu Thr Ser Arg Phe Thr Lys Leu Leu Leu Lys Leu
               580                 585                 590
   Pro Asp Leu Arg Thr Leu Asn Asn Met His Ser Glu Lys Leu Leu Ser
           595                 600                 605
   Phe Arg Val Asp Ala Gln
       610
<210> 11
   <211> 4346
   <212> DNA
   <213> Homo sapiens
<220>
   <221> CDS
   <222> (265)..(2004)
   <223>
<400> 11
   ggctgcagga agccgccgcg ccgccgcttt tgttgtcagg gacccagcga ggagcgccgc      60
   tcgccggccg ccgccaccct ctctcgctgc agcctgctgt gcgctgcacg gcctggggcc    120
   cgggagcccc gcccgctctg cccatgaggg ggccccgcga ccaccgctgc ttccagcccg    180
   gggcggcgcg gcgctgaggc ggcggcggcg gcgctgcccc ctctgcggga agcgggcggc    240
   cccggccgcc tccgcgaggg cacc atg gag gtg aat gca gga ggt gtg att       291
                              Met Glu Val Asn Ala Gly Gly Val Ile
                              1               5
   gcc tat atc agt tct tcc agc tca gcc tca agc cat gcc tct tgt cac      339
   Ala Tyr Ile Ser Ser Ser Ser Ser Ala Ser Ser His Ala Ser Cys His
   10                  15                  20                  25
   agt gag ggt tct gag aat agt ttc cag tcc tcc tcc tct tct gtt cca      387
   Ser Glu Gly Ser Glu Asn Ser Phe Gln Ser Ser Ser Ser Ser Val Pro
                   30                  35                  40
   tct tct cca aat agc tct aat tct gat acc aat ggt aat ccc aag aat      435
   Ser Ser Pro Asn Ser Ser Asn Ser Asp Thr Asn Gly Asn Pro Lys Asn
               45                  50                  55
   ggt gat ctc gcc aat att gaa ggc atc ttg aag aat gat cga ata gat      483
   Gly Asp Leu Ala Asn Ile Glu Gly Ile Leu Lys Asn Asp Arg Ile Asp
           60                  65                  70
   tgt tct atg aaa aca agc aaa tcg agt gca cct ggg atg aca aaa agt      531
   Cys Ser Met Lys Thr Ser Lys Ser Ser Ala Pro Gly Met Thr Lys Ser
       75                  80                  85
   cat agt ggt gtg aca aaa ttt agt ggc atg gtt cta ctg tgt aaa gtc      579
   His Ser Gly Val Thr Lys Phe Ser Gly Met Val Leu Leu Cys Lys Val
   90                  95                  100                 105
   tgt ggg gat gtg gcg tca gga ttc cac tat gga gtt cat gct tgc gaa      627
   Cys Gly Asp Val Ala Ser Gly Phe His Tyr Gly Val His Ala Cys Glu
                   110                 115                 120
   ggc tgt aag ggt ttc ttt cgg aga agt att caa caa aac atc cag tac      675
   Gly Cys Lys Gly Phe Phe Arg Arg Ser Ile Gln Gln Asn Ile Gln Tyr
               125                 130                 135
   aag aag tgc ctg aag aat gaa aac tgt tct ata atg aga atg aat agg      723
   Lys Lys Cys Leu Lys Asn Glu Asn Cys Ser Ile Met Arg Met Asn Arg
           140                 145                 150
   aac aga tgt cag caa tgt cgc ttc aaa aag tgt ctg tct gtt gga atg      771
   Asn Arg Cys Gln Gln Cys Arg Phe Lys Lys Cys Leu Ser Val Gly Met
       155                 160                 165
   tca aga gat gct gtt cgg ttt ggt cgt att cct aag cgt gaa aaa cag      819
   Ser Arg Asp Ala Val Arg Phe Gly Arg Ile Pro Lys Arg Glu Lys Gln
   170                 175                 180                 185
   agg atg cta att gaa atg caa agt gca atg aag acc atg atg aac agc      867
   Arg Met Leu Ile Glu Met Gln Ser Ala Met Lys Thr Met Met Asn Ser
                   190                 195                 200
   cag ttc agt ggt cac ttg caa aat gac aca tta gta gaa cat cat gaa      915
   Gln Phe Ser Gly His Leu Gln Asn Asp Thr Leu Val Glu His His Glu
               205                 210                 215
   cag aca gcc ttg cca gcc cag gaa cag ctg cga ccc aag ccc caa ctg      963
   Gln Thr Ala Leu Pro Ala Gln Glu Gln Leu Arg Pro Lys Pro Gln Leu
           220                 225                 230
   gag caa gaa aac atc aaa agc tct tct cct cca tct tct gat ttt gca     1011
   Glu Gln Glu Asn Ile Lys Ser Ser Ser Pro Pro Ser Ser Asp Phe Ala
       235                 240                 245
   aag gaa gaa gtg att ggc atg gtg acc aga gct cac aag gat acc ttt     1059
   Lys Glu Glu Val Ile Gly Met Val Thr Arg Ala His Lys Asp Thr Phe
   250                 255                 260                 265
   atg tat aat caa gag cag caa gaa aac tca gct gag agc atg cag ccc     1107
   Met Tyr Asn Gln Glu Gln Gln Glu Asn Ser Ala Glu Ser Met Gln Pro
                   270                 275                 280
   aag aga gga gaa cgg att cgc aag aac atg gag caa tat aat tta aat     1155
   Lys Arg Gly Glu Arg Ile Arg Lys Asn Met Glu Gln Tyr Asn Leu Asn
               285                 290                 295
   cat gat cat tgc ggc aat ggg ctt agc agc cat ttt ccc tgt agt gag     1203
   His Asp His Cys Gly Asn Gly Leu Ser Ser His Phe Pro Cys Ser Glu
           300                 305                 310
   agc cag cag cat ctc aat gga cag ttc aaa ggg agg aat ata atg cat     1251
   Ser Gln Gln His Leu Asn Gly Gln Phe Lys Gly Arg Asn Ile Met His
       315                 320                 325
   tac cca aat ggt cat gcc att tgt att gca aat gga cat tgt atg aac     1299
   Tyr Pro Asn Gly His Ala Ile Cys Ile Ala Asn Gly His Cys Met Asn
   330                 335                 340                 345
   ttc tcc aat gct tat act caa aga gta tgt gat aga gtt ccg ata gat     1347
   Phe Ser Asn Ala Tyr Thr Gln Arg Val Cys Asp Arg Val Pro Ile Asp
                   350                 355                 360
   gga ttt tct cag aat gag aac aag aat agt tac ctg tgc aac act gga     1395
   Gly Phe Ser Gln Asn Glu Asn Lys Asn Ser Tyr Leu Cys Asn Thr Gly
               365                 370                 375
   gga aga atg cat ctg gtt tgt cca atg agt aag tct cca tat gtg gat     1443
   Gly Arg Met His Leu Val Cys Pro Met Ser Lys Ser Pro Tyr Val Asp
           380                 385                 390
   cct cat aaa tca gga cat gaa atc tgg gaa gaa ttt tcg atg agc ttc     1491
   Pro His Lys Ser Gly His Glu Ile Trp Glu Glu Phe Ser Met Ser Phe
       395                 400                 405
   act cca gca gtg aaa gaa gtg gtg gaa ttt gca aag cgt att cct ggg     1539
   Thr Pro Ala Val Lys Glu Val Val Glu Phe Ala Lys Arg Ile Pro Gly
   410                 415                 420                 425
   ttc aga gat ctc tct cag cat gac cag gtc aac ctt tta aag gct ggg     1587
   Phe Arg Asp Leu Ser Gln His Asp Gln Val Asn Leu Leu Lys Ala Gly
                   430                 435                 440
   act ttt gag gtt tta atg gta cgg ttc gca tca tta ttt gat gca aag     1635
   Thr Phe Glu Val Leu Met Val Arg Phe Ala Ser Leu Phe Asp Ala Lys
               445                 450                 455
   gaa cgt act gtc acc ttt tta agt gga aag aaa tat agt gtg gat gat      1683
   Glu Arg Thr Val Thr Phe Leu Ser Gly Lys Lys Tyr Ser Val Asp Asp
           460                 465                 470
   tta cac tca atg gga gca ggg gat ctg cta aac tct atg ttt gaa ttt      1731
   Leu His Ser Met Gly Ala Gly Asp Leu Leu Asn Ser Met Phe Glu Phe
       475                 480                 485
   agt gag aag cta aat gcc ctc caa ctt agt gat gaa gag atg agt ttg      1779
   Ser Glu Lys Leu Asn Ala Leu Gln Leu Ser Asp Glu Glu Met Ser Leu
   490                 495                 500                 505
   ttt aca gct gtt gtc ctg gta tct gca gat cga tct gga ata gaa aac      1827
   Phe Thr Ala Val Val Leu Val Ser Ala Asp Arg Ser Gly Ile Glu Asn
                   510                 515                 520
   gtc aac tct gtg gag gct ttg cag gaa act ctc att cgt gca cta agg      1875
   Val Asn Ser Val Glu Ala Leu Gln Glu Thr Leu Ile Arg Ala Leu Arg
               525                 530                 535
   acc tta ata atg aaa aac cat cca aat gag gcc tct att ttt aca aaa      1923
   Thr Leu Ile Met Lys Asn His Pro Asn Glu Ala Ser Ile Phe Thr Lys
           540                 545                 550
   ctg ctt cta aag ttg cca gat ctt cga tct tta aac aac atg cac tct      1971
   Leu Leu Leu Lys Leu Pro Asp Leu Arg Ser Leu Asn Asn Met His Ser
       555                 560                 565
   gag gag ctc ttg gcc ttt aaa gtt cac cct taa ggcctttgtt tatttaaaca   2024
   Glu Glu Leu Leu Ala Phe Lys Val His Pro
   570                 575
   tgaactgatg gtaactgtac attttgtgct aaaatgcata tttatatgtg tataccatat   2084
   gtggagatag aaaagacctt taagacaata aaagattgta ggctatctct gtaatcatgc   2144
   aatagctgtt cggattgaga actcttcagc catgattaga cgttgactgc atctccctga   2204
   tagaccaatc agctgtgtcg cacttaaact ggagaagtta cactgaagta taatcacact   2264
   gaatgttata ctttttcatc tgccaaaacc aaaaaccatt ttgatctccc tgtggttatc   2324
   aatataacgc acaatcacaa gtgtatgagg acttagaaat taatcctttg tggtaggagt   2384
   tctgttgaat gatggaaatc ttattactac cacaagacta tttgttctgg taattggaga   2444
   cttcgggatt taggagatct ccatgtctgt atttactcta ccactgctaa agtgtgtggt   2504
   cctgggtagt ttacttgctt gcggaaaatg agaattgatg gtgtccccaa tgccccacct   2564
   cacagagtta ctaaaaaatg tctgtaaagc atatttacct cttgggagat aggcactatg   2624
   taaataaggt aaaatttctg ttattacaat tattcataat aatattcttt tcttatttct   2684
   aagcctttct gggaaatcat ttcagtccac accaaccata ttattcaggg ttcctgccat   2744
   atgtgtgggg tatcctactg atacacacgt attcaaagtt tatgggtaca acaaagacat   2804
   agtacatgta cataatatgt atgtgaatat agttaaatat atttcttcac aatattttaa   2864
   actgtgaaga actttatcat acaggaaact taaaacaaga ggtgtcaaaa gacccaaatt   2924
   aggtgcattt tacttgttta tgatggcata accattgctt taaaatgttt agacagtaga   2984
   atattgaatt tatgctctat ttttgtttat ttaagcaaca cttaatgtaa aagtgcaaca   3044
   ggcaattgaa tccaaatttc aacgacaaaa aaaaaaaaca tgtattttag agttcatctt   3104
   tggcaaaatc tttggttcag ggtactagtt gtttaaaagt tgattcatat tcttaccttg   3164
   tgctgagaaa ggttgcattg ctgcccctta tacacatgct gcagcttgat gttaaagaat   3224
   ttttattctt tctgaagaac taattaatgt ttaaagcaac tgtttaatat gatggcatgt   3284
   gtgtgtgtgc gtgcgtgtgt atgttctgag tccacttctt ttttcctaaa taacactaca   3344
   gggattttgt catattagat ttaatttata atttgaaaaa tcatctagtg tgtgacctac   3404
   aggcttagaa atggtatagt caaagacatt ttatccacat ttctaatagt ggacttgatt   3464
   aagtagataa gatcagcatc tgtttatggt attaggagaa atagccaaag ttgaggattt   3524
   tatgtatgtt ttcctgttta cctggaaaat agcaattaat tggatttttt ggtaaagatt   3584
   gccttctgta taatgtttgg attatataaa attgcaaaaa tgataacagc ccgctttact   3644
   gtactaagcc tgttactctc atgacgtgtg agcagaatgc cttattttgt aatcttgttt   3704
   aacttgttgc tactgggact tgatttactg tggcactagt taagtaagtt aaaaaaaagt   3764
   taaaccctct cattattaaa gaggaaaggc gatggtgatg tctgtagtac aatataaacc   3824
   ataattgtga tttaccttaa gtaggtataa ctcttatggg atatacagta tagtttttgt   3884
   gaatctttac atgatagcat tatcttttta taattttttt tcctaagata aacaaatgca   3944
   tagttttctt ctatgggtga tagaaacagc tttttgaagt aatgaaaacc tcaaaagatc   4004
   atgttgattc ttaatttttg ccttttgcat aagcctcttt ataacatgta tctttaaaac    4064
   aattaagtct ttaggaatgt gtaaccagaa ctatgttagt attgcttata aaactttagt   4124
   taggttcaat atatacatat atacatctct atataggtat atagatttgc attttgtctt   4184
   gtaaaatttt atttgaataa attcttcctg taggtaatgg gaaacaaaat taatagttca   4244
   tatgtcactc atagcatttc tatatttgaa agtagcccaa tataaaactt ttgattctaa   4304
   aattaaacca gcagcctatt acaagcaaaa aaaaaaaaaa aa                      4346
<210> 12
   <211> 579
   <212> PRT
   <213> Homo sapiens
<400> 12
   Met Glu Val Asn Ala Gly Gly Val Ile Ala Tyr Ile Ser Ser Ser Ser
   1               5                   10                  15
   Ser Ala Ser Ser His Ala Ser Cys His Ser Glu Gly Ser Glu Asn Ser
               20                  25                  30
   Phe Gln Ser Ser Ser Ser Ser Val Pro Ser Ser Pro Asn Ser Ser Asn
           35                  40                  45
   Ser Asp Thr Asn Gly Asn Pro Lys Asn Gly Asp Leu Ala Asn Ile Glu
       50                  55                  60
   Gly Ile Leu Lys Asn Asp Arg Ile Asp Cys Ser Met Lys Thr Ser Lys
   65                  70                  75                  80
   Ser Ser Ala Pro Gly Met Thr Lys Ser His Ser Gly Val Thr Lys Phe
                   85                  90                  95
   Ser Gly Met Val Leu Leu Cys Lys Val Cys Gly Asp Val Ala Ser Gly
               100                 105                 110
   Phe His Tyr Gly Val His Ala Cys Glu Gly Cys Lys Gly Phe Phe Arg
           115                 120                 125
   Arg Ser Ile Gln Gln Asn Ile Gln Tyr Lys Lys Cys Leu Lys Asn Glu
       130                 135                 140
   Asn Cys Ser Ile Met Arg Met Asn Arg Asn Arg Cys Gln Gln Cys Arg
   145                 150                 155                 160
   Phe Lys Lys Cys Leu Ser Val Gly Met Ser Arg Asp Ala Val Arg Phe
                   165                 170                 175
   Gly Arg Ile Pro Lys Arg Glu Lys Gln Arg Met Leu Ile Glu Met Gln
               180                 185                 190
   Ser Ala Met Lys Thr Met Met Asn Ser Gln Phe Ser Gly His Leu Gln
           195                 200                 205
   Asn Asp Thr Leu Val Glu His His Glu Gln Thr Ala Leu Pro Ala Gln
       210                 215                 220
   Glu Gln Leu Arg Pro Lys Pro Gln Leu Glu Gln Glu Asn Ile Lys Ser
   225                 230                 235                 240
   Ser Ser Pro Pro Ser Ser Asp Phe Ala Lys Glu Glu Val Ile Gly Met
   245 250 255
   Val Thr Arg Ala His Lys Asp Thr Phe Met Tyr Asn Gln Glu Gln Gln
   260 265 270
   Glu Asn Ser Ala Glu Ser Met Gln Pro Lys Arg Gly Glu Arg Ile Arg
   275 280 285
   Lys Asn Met Glu Gln Tyr Asn Leu Asn His Asp His Cys Gly Asn Gly
   290 295 300
   Leu Ser Ser His Phe Pro Cys Ser Glu Ser Gln Gln His Leu Asn Gly
   305 310 315 320
   Gln Phe Lys Gly Arg Asn Ile Met His Tyr Pro Asn Gly His Ala Ile
   325 330 335
   Cys Ile Ala Asn Gly His Cys Met Asn Phe Ser Asn Ala Tyr Thr Gln
   340 345 350
   Arg Val Cys Asp Arg Val Pro Ile Asp Gly Phe Ser Gln Asn Glu Asn
   355 360 365
   Lys Asn Ser Tyr Leu Cys Asn Thr Gly Gly Arg Met His Leu Val Cys
   370 375 380
   Pro Met Ser Lys Ser Pro Tyr Val Asp Pro His Lys Ser Gly His Glu
   385 390 395 400
   Ile Trp Glu Glu Phe Ser Met Ser Phe Thr Pro Ala Val Lys Glu Val
   405 410 415
   Val Glu Phe Ala Lys Arg Ile Pro Gly Phe Arg Asp Leu Ser Gln His
   420 425 430
   Asp Gln Val Asn Leu Leu Lys Ala Gly Thr Phe Glu Val Leu Met Val
   435 440 445
   Arg Phe Ala Ser Leu Phe Asp Ala Lys Glu Arg Thr Val Thr Phe Leu
   450 455 460
   Ser Gly Lys Lys Tyr Ser Val Asp Asp Leu His Ser Met Gly Ala Gly
   465 470 475 480
   Asp Leu Leu Asn Ser Met Phe Glu Phe Ser Glu Lys Leu Asn Ala Leu
                   485                 490                 495
   Gln Leu Ser Asp Glu Glu Met Ser Leu Phe Thr Ala Val Val Leu Val
               500                 505                 510
   Ser Ala Asp Arg Ser Gly Ile Glu Asn Val Asn Ser Val Glu Ala Leu
           515                 520                 525
   Gln Glu Thr Leu Ile Arg Ala Leu Arg Thr Leu Ile Met Lys Asn His
       530                 535                 540
   Pro Asn Glu Ala Ser Ile Phe Thr Lys Leu Leu Leu Lys Leu Pro Asp
   545                 550                 555                 560
   Leu Arg Ser Leu Asn Asn Met His Ser Glu Glu Leu Leu Ala Phe Lys
                   565                 570                 575
   Val His Pro
<210> 13
   <211> 1478
   <212> DNA
   <213> Homo sapiens
<220>
   <221> CDS
   <222> (338)..(1315)
   <223>
<400> 13
   cagattttgc aagagccagg ctcgcccacc ttgtagaagg agcgccttga gtcccctctc      60
   accctcggtt gcaaagagcc gaccgcttga tctggacacc ccctcgccca gattgcatga     120
   tctcccggga ccctcttgag ttgcacgttt ctgcaccgag gacctcaaat ccccgtcgct     180
   cctaggattt gcagcgttct ggatactgga gggttgcagg ctacactcgc ccgcccctgg     240
   gcagacactc gtccaaacca ctggagtgtg ctggtgactg gcaggccagc ccttcgcctc     300
   tccatgaacc cgtgagcctg ggggcaggtg ccaggcg atg gcg cgg cct gtg agc      355
                                            Met Ala Arg Pro Val Ser
                                            1               5
   gac agg acc ccg gcc cct ctg ctg ctg ggc ggc ccg gcc ggg aca ccc       403
   Asp Arg Thr Pro Ala Pro Leu Leu Leu Gly Gly Pro Ala Gly Thr Pro
               10                  15                  20
   cct ggc ggg gga gcg ctg ctt ggg ttg cgg agc ctt ctg cag ggg acc       451
   Pro Gly Gly Gly Ala Leu Leu Gly Leu Arg Ser Leu Leu Gln Gly Thr
               25                  30                  35
   agc aag ccc aaa gag ccg gcc agc tgt ctc ctg aag gaa aag gag cgc        499
   Ser Lys Pro Lys Glu Pro Ala Ser Cys Leu Leu Lys Glu Lys Glu Arg
       40                  45                  50
   aag gcg gcc ctg cct gca gcc aca acc cct ggg cca ggc ctg gag act        547
   Lys Ala Ala Leu Pro Ala Ala Thr Thr Pro Gly Pro Gly Leu Glu Thr
   55                  60                  65                  70
   gcg ggc ccg gcg gat gcc ccg gct ggg gca gtg gtg ggc gga ggg tcc        595
   Ala Gly Pro Ala Asp Ala Pro Ala Gly Ala Val Val Gly Gly Gly Ser
                   75                  80                  85
   ccg cgg ggg cgc ccg ggg ccg gtg ccc gcc ccg ggt ctg ttg gcg cca        643
   Pro Arg Gly Arg Pro Gly Pro Val Pro Ala Pro Gly Leu Leu Ala Pro
               90                  95                  100
   ctg ctg tgg gag cgc acg ctg ccg ttc ggc gat gtg gag tac gta gac        691
   Leu Leu Trp Glu Arg Thr Leu Pro Phe Gly Asp Val Glu Tyr Val Asp
           105                 110                 115
   ctg gac gcc ttc ctg ctg gag cac ggg ctc ccg ccc agc ccg ccg ccc        739
   Leu Asp Ala Phe Leu Leu Glu His Gly Leu Pro Pro Ser Pro Pro Pro
       120                 125                 130
   ccc ggt ggc ccg tcg ccg gag ccg tcg ccc gcg cgg acg ccc gca ccc        787
   Pro Gly Gly Pro Ser Pro Glu Pro Ser Pro Ala Arg Thr Pro Ala Pro
   135                 140                 145                 150
   tcc cca ggg ccg ggt tcg tgc ggc tcg gct tcc ccc cgc tcc tct cct        835
   Ser Pro Gly Pro Gly Ser Cys Gly Ser Ala Ser Pro Arg Ser Ser Pro
                   155                 160                 165
   ggg cac gcc ccc gcc cgg gct gcc ctc ggg acc gcc agc ggc cac cgc        883
   Gly His Ala Pro Ala Arg Ala Ala Leu Gly Thr Ala Ser Gly His Arg
               170                 175                 180
   gca ggc ctg acc tct cgg gac aca ccc agc cct gtg gac cca gac acc        931
   Ala Gly Leu Thr Ser Arg Asp Thr Pro Ser Pro Val Asp Pro Asp Thr
           185                 190                 195
   gtg gag gtg ttg atg acc ttt gaa ccc gac cca gct gat ctt gcc cta        979
   Val Glu Val Leu Met Thr Phe Glu Pro Asp Pro Ala Asp Leu Ala Leu
       200                 205                 210
   tca agc att cct ggc cac gag acc ttt gac cct cga aga cat cgc ttc       1027
   Ser Ser Ile Pro Gly His Glu Thr Phe Asp Pro Arg Arg His Arg Phe
   215                 220                 225                 230
   tca gaa gag gaa ctt aag ccc cag cca atc atg aag aag gca aga aaa       1075
   Ser Glu Glu Glu Leu Lys Pro Gln Pro Ile Met Lys Lys Ala Arg Lys
                   235                 240                 245
   atc cag gtg ccg gag gag cag aag gat gag aaa tac tgg agc cgg cgg       1123
   Ile Gln Val Pro Glu Glu Gln Lys Asp Glu Lys Tyr Trp Ser Arg Arg
               250                 255                 260
   tac aag aac aac gag gca gcc aag cgg tcc cgt gac gcc cgg cgg ctc       1171
   Tyr Lys Asn Asn Glu Ala Ala Lys Arg Ser Arg Asp Ala Arg Arg Leu
           265                 270                 275
   aag gag aac cag ata tcg gtg cgg gcg gcc ttc ctg gag aag gag aac      1219
   Lys Glu Asn Gln Ile Ser Val Arg Ala Ala Phe Leu Glu Lys Glu Asn
       280                 285                 290
   gcc ctg ctg cgg cag gaa gtt gtg gcc gtg cgc cag gag ctg tcc cac      1267
   Ala Leu Leu Arg Gln Glu Val Val Ala Val Arg Gln Glu Leu Ser His
   295                 300                 305                 310
   tac cgc gcc gtg ctg tcc cga tac cag gcc cag cac ggg gcc ctg tga      1315
   Tyr Arg Ala Val Leu Ser Arg Tyr Gln Ala Gln His Gly Ala Leu
                   315                 320                 325
   ggctgcccca catccccacc tggcggagct ctcctccgcc ttgctgagac ttacgccctg    1375
   ttcccttcct gccctgtggc ccacgggccg gccagctggg tgccccaggg acgtgataat    1435
   gcagataaat acatttatat ttttaagaaa aaaaaaaaaa aaa                      1478
<210> 14
   <211> 325
   <212> PRT
   <213> Homo sapiens
<400> 14
   Met Ala Arg Pro Val Ser Asp Arg Thr Pro Ala Pro Leu Leu Leu Gly
   1               5                   10                  15
   Gly Pro Ala Gly Thr Pro Pro Gly Gly Gly Ala Leu Leu Gly Leu Arg
               20                  25                  30
   Ser Leu Leu Gln Gly Thr Ser Lys Pro Lys Glu Pro Ala Ser Cys Leu
           35                  40                  45
   Leu Lys Glu Lys Glu Arg Lys Ala Ala Leu Pro Ala Ala Thr Thr Pro
       50                  55                  60
   Gly Pro Gly Leu Glu Thr Ala Gly Pro Ala Asp Ala Pro Ala Gly Ala
   65                  70                  75                  80
   Val Val Gly Gly Gly Ser Pro Arg Gly Arg Pro Gly Pro Val Pro Ala
                   85                  90                  95
   Pro Gly Leu Leu Ala Pro Leu Leu Trp Glu Arg Thr Leu Pro Phe Gly
               100                 105                 110
   Asp Val Glu Tyr Val Asp Leu Asp Ala Phe Leu Leu Glu His Gly Leu
           115                 120                 125
   Pro Pro Ser Pro Pro Pro Pro Gly Gly Pro Ser Pro Glu Pro Ser Pro
       130                 135                 140
   Ala Arg Thr Pro Ala Pro Ser Pro Gly Pro Gly Ser Cys Gly Ser Ala
   145                 150                 155                 160
   Ser Pro Arg Ser Ser Pro Gly His Ala Pro Ala Arg Ala Ala Leu Gly
                   165                 170                 175
   Thr Ala Ser Gly His Arg Ala Gly Leu Thr Ser Arg Asp Thr Pro Ser
               180                 185                 190
   Pro Val Asp Pro Asp Thr Val Glu Val Leu Met Thr Phe Glu Pro Asp
           195                 200                 205
   Pro Ala Asp Leu Ala Leu Ser Ser Ile Pro Gly His Glu Thr Phe Asp
       210                 215                 220
   Pro Arg Arg His Arg Phe Ser Glu Glu Glu Leu Lys Pro Gln Pro Ile
   225                 230                 235                 240
   Met Lys Lys Ala Arg Lys Ile Gln Val Pro Glu Glu Gln Lys Asp Glu
                   245                 250                 255
   Lys Tyr Trp Ser Arg Arg Tyr Lys Asn Asn Glu Ala Ala Lys Arg Ser
               260                 265                 270
   Arg Asp Ala Arg Arg Leu Lys Glu Asn Gln Ile Ser Val Arg Ala Ala
           275                 280                 285
   Phe Leu Glu Lys Glu Asn Ala Leu Leu Arg Gln Glu Val Val Ala Val
       290                 295                 300
   Arg Gln Glu Leu Ser His Tyr Arg Ala Val Leu Ser Arg Tyr Gln Ala
   305                 310                 315                 320
   Gln His Gly Ala Leu
                   325
<210> 15
   <211> 26
   <212> DNA
   <213> Artificial
<220>
   <223> Forward Primer for the Per3 gene
<400> 15
   ctacctgcac cctgaagatc gttctc 26
<210> 16
   <211> 30
   <212> DNA
   <213> Artificial
<220>
   <223> Reverse primer for the Per3 gene
<400> 16
   ctggaatcca gtatgatgta gtctccgttt 30
<210> 17
   <211> 20
   <212> DNA
   <213> Artificial
<220>
   <223> Forward primer fot the Per2 gene
<400> 17
   gcttcccttg tggagcatgt 20
<210> 18
   <211> 20
   <212> DNA
   <213> Artificial
<220>
   <223> Reverse primer for the Per2 gene
<400> 18
   ttgaccaggt agggcgtcat 20
<210> 19
   <211> 30
   <212> DNA
   <213> Artificial
<220>
   <223> Forward primer for the Bmall gene
<400> 19
   catgcaacgc aatgtccagg aaattagata 30
<210> 20
   <211> 30
   <212> DNA
   <213> Artificial
<220>
   <223> Reverse primer for the Bmall gene
<400> 20
   cctgagaatg aggtgtttca attcatcgtc 30
<210> 21
   <211> 30
   <212> DNA
   <213> Artificial
<220>
   <223> Forward primer for the Npas gene
<400> 21
   cgggaccagt tcaatgttct catcaaagag 30
<210> 22
   <211> 29
   <212> DNA
   <213> Artificial
<220>
   <223> Reverse primer for the Npas gene
<400> 22
   gtcttgctga atgtcacaga tttccgttt 29
<210> 23
   <211> 22
   <212> DNA
   <213> Artificial
<220>
   <223> Forward primer for the Nr1d1 gene
<400> 23
   gctcagtgcc atgttcgact tc 22
<210> 24
   <211> 20
   <212> DNA
   <213> Artificial
<220>
   <223> Reverse primer for the Nr1d1 gene
<400> 24
   aagtctccaa gggccggttc 20
<210> 25
   <211> 30
   <212> DNA
   <213> Artificial
<220>
   <223> Forward primer for the Nr1d2 gene
<400> 25
   tccagtacaa gaagtgcctg aagaatgaaa 30
<210> 26
   <211> 26
   <212> DNA
   <213> Artificial
<220>
   <223> Reverse primer for the Nr1d2 gene
<400> 26
   cacgcttagg aatacgacca aaccga 26
<210> 27
   <211> 22
   <212> DNA
   <213> Artificial
<220>
   <223> Forward primer for the Dbp gene
<400> 27
   tcgttgttct tgtaccgccg gc 22
<210> 28
   <211> 28
   <212> DNA
   <213> Artificial
<220>
   <223> Reverse primer for the Dbp gene
<400> 28
   ccagctgatc ttgccctatc aagcattc 28
<210> 29
   <211> 20
   <212> DNA
   <213> Artificial
<220>
   <223> Forward primer for the 18s rRNA
<400> 29
   cgccgctaga ggtgaaattc 20
<210> 30
   <211> 21
   <212> DNA
   <213> Artificial
<220>
   <223> Reverse primer for the 18s rRNA
<400> 30
   cgaacctccg actttcgttc t 21